# EUROPEAN PATENT APPLICATION

(11) **EP 4 567 129 A2**
(43) Date of publication of application: **11.06.2025**
(21) Application number: 25158902.4
(22) Date of filing: 02.02.2023
(51) Int. Cl.: C12Q 1/6874

(54) **MULTIFUNCTIONAL PRIMERS FOR PAIRED SEQUENCING READS**

(30) Priority: 02.02.2022 US 202263267466 P
(62) Divisional of application: 23718537.6
(71) Applicant: Guardant Health, Inc., Palo Alto, CA 94304 (US)
(72) Inventor: KENNEDY, Andrew, Palo Alto, 94304 (US)
(74) Representative: J A Kemp LLP

(57) **Abstract**

The invention provides methods of generating forward and reverse reads of an immobilized single-stranded target nucleic acid using multifunctional primers. A multifunctional primer serves to initiate separate syntheses of first and second complementary strands to the single-stranded target nucleic acid, and to tether the first complementary strand to the immobilized target nucleic acid. The first complementary strand serves to provide a primer binding site and template for an extension product to generate a reverse sequencing read. The second complementary strand serves to displace the first complementary strand from duplexing with the target nucleic acid. The first or second complementary strand can provide a forward sequence read.

## Description

### CROSS REFERENCE TO RELATED APPLICATION

The present application claims the benefit of U.S. Provisional Application No. 63/267,466, filed February 2, 2022, incorporated by reference in its entirety for all purposes.

### BACKGROUND

The original methods in DNA sequencing were Sanger dideoxy synthesis and Maxam-Gilbert chemical cleavage. Maxam-Gilbert is based on chemical modification of DNA and subsequent cleavage of the DNA backbone at sites adjacent to the modified nucleotides. Sanger sequencing uses specific chain-terminating nucleotides (dideoxy nucleotides) that lack a 3'-OH group, resulting in termination of the growing DNA chain at that position. The ddNTPs are radioactively or fluorescently labeled for detection in sequencing gels or automated sequencing machines.

A need for higher throughput sequencing of large genomes at lower cost triggered development of next generation sequencing techniques. Many next generation techniques distribute individual DNA molecules to be sequenced to millions of separate beads, wells or chambers, or tether them to addressable locations on a planar support. The DNA molecules, often following amplification, are subjected to DNA synthesis reactions in which labeled nucleotides, or chemical reactions based on the incorporation of a particular nucleotide, can be imaged or otherwise detected.

Although next generation methods generate a much higher greater amount of sequence at lower cost than the original methods, they often rely on much shorter reads (e.g., up to about 300-500 bases). Further, they often have a higher error rate than the original methods.

### SUMMARY OF THE CLAIMED INVENTION

The invention provides a method of obtaining paired sequencing reads of a target nucleic acid. The method comprises (a) contacting a single-stranded target nucleic acid flanked at its 5' end by a 5' adapter immobilized to a support and at its 3' end by a 3' adapter with a forward primer comprising a 3' target binding region and a 5' stem-loop to form a reaction mix, wherein the target binding region binds the 3' adapter; (b) ligating a free 5' end of the stem loop to the 3' adapter; (c) conducting an extension reaction, wherein the target binding region primes synthesis of a first complementary strand to the target nucleic acid and optionally provides a sequencing read of the target nucleic acid; (d) contacting the reaction mix with a loop primer that anneals to the loop region of the stem loop; (e) conducting an extension reaction, wherein the loop primer primes synthesis of a second complementary strand to the target nucleic acid, and the first complementary strand is displaced from duplexing with the target nucleic acid; (f) contacting the reaction mix with a reverse primer, which anneals to a complement of the 5' adapter at the 3' end of the displaced first complementary strand; and (g) conducting a sequencing-by-extension reaction, wherein the reverse primer primes synthesis of a strand complementary to the displaced first complementary strand and provides a sequencing read of the displaced first complementary strand.

Optionally, the method further comprises before step (a) contacting the single-stranded target nucleic acid with a second forward primer comprising a target binding region, which binds to a complementary site in the 3' adapter; conducting a sequencing-by-extension reaction wherein the target binding region of the second forward primer primes synthesis of a further complementary strand of the target nucleic acid and provides a sequencing read of the target nucleic acid; and displacing the further complementary strand from duplexing with the target nucleic acid. Optionally, step (c) is a sequencing-by-extension reaction that provides a sequence read of the target nucleic acid. Optionally, step (c) is performed before step (b).

The invention further provides a method of obtaining paired sequencing reads of a target nucleic acid. The method comprises (a) contacting a single-stranded target nucleic acid flanked at its 5' end by a 5' adapter immobilized to a support and at its 3' end by a 3' adapter with a forward primer comprising a 3' target binding region and a 5' stem loop to form a reaction mix, wherein the target binding region binds the 3' adapter and wherein the target binding region comprises a cleavable site; (b) ligating a free 5' end of the stem loop to the 3' adapter; (c) conducting a sequencing-by-extension reaction, wherein the target binding region primes synthesis of a first complementary strand to the target nucleic acid and provides a sequencing read of the target nucleic acid; cleaving the cleavable site, and conducting a further extension reaction wherein the remainder of the target binding region after cleavage primes synthesis of a further complementary strand to the target nucleic acid; (d) contacting the reaction mix with a loop primer that anneals to the loop region of the stem loop; (e) conducting an extension reaction wherein the loop primer primes synthesis of a second complementary strand to the target nucleic acid, which displaces the further complementary strand from duplexing with the target nucleic acid; (f) contacting the reaction mix with a reverse primer, which anneals to a complement of the 5' adapter at the 3' end of the displaced further complementary strand; (g) conducting a sequencing-by-extension reaction, wherein the reverse primer primes synthesis of a strand complementary to the displaced further complementary strand and provides a sequencing read of the displaced further complementary strand.

The invention further provides a method of obtaining paired sequencing reads from a target nucleic acid. The method comprises (a) contacting a single-stranded target nucleic acid flanked at its 5' end by a 5' adapter immobilized to a support and at its 3' end by a 3' adapter with a forward hairpin primer comprising a 3' target binding region and a 5' stem loop and a forward linear primer to form a reaction mix, wherein the target binding region of the forward hairpin stem primer and the linear forward primer bind the 3' adapter with the stem loop primer between the linear primer and the 3' end; (b) ligating a free 5' end of the step loop to the 3' end of the adapter molecule at the 3' end of the target nucleic acid; (c) conducting a sequencing-by-extension reaction, wherein the linear primer primes synthesis of a first complementary strand to the target nucleic acid and provides a sequencing read of the target nucleic acid; and conducting a further extension reaction wherein the target binding region of the forward stem loop primer primes synthesis of a further complementary strand to the target nucleic acid; (d) contacting the reaction mix with a loop primer that anneals to the loop region of the stem loop; (e) conducting an extension reaction wherein the loop primer primes synthesis of a second complementary strand to the target nucleic acid, which displaces the further complementary strand from duplexing with the target nucleic acid; (f) contacting the reaction mix with a reverse primer, which anneals with a complement of the 5' adapter at the 3' end of the displaced further complementary strand; and (g) conducting a sequencing-by-extension reaction, wherein the reverse primer primes synthesis of a strand complementary to the displaced further complementary strand and provides a sequencing read of the displaced further complementary strand. Optionally, the stem loop forward primer contains an extension-blocked nucleotide and the method further comprises removing the block before extending the stem loop forward primer.

The invention further provides a method of obtaining paired sequencing reads from a target nucleic acid. The method includes (a) contacting a single-stranded target nucleic acid flanked at its 5' end by a 5' adapter immobilized to a support and at its 3' end by a 3' adapter with a forward primer comprising first and second 3' target binding regions flanking an intervening region to form a reaction mix, wherein the first and second target binding regions bind complementary sites in the 3' adapter molecule, the respective sites being distal and proximal to the 3' end of the 3' adapter; (b) conducting an extension reaction, wherein the first target binding region primes synthesis of a first complementary strand to the target nucleic acid and optionally provides a sequencing read of the target nucleic acid; (c) conducting a second extension reaction wherein the second target binding region primes synthesis of a second complementary strand to the target nucleic acid, which displaces the complementary strand from duplexing with the target nucleic acid; (d) contacting the reaction mix with a reverse primer, which anneals with a complement of the 5' adapter at the 3' end of the displaced first complementary strand; and (e) conducting a sequencing-by-extension reaction, wherein the reverse primer primes synthesis of a strand complementary to the displaced first complementary strand and provides a sequencing read of the displaced first complementary strand. Optionally, the method further comprises before step (a) contacting the single-stranded target nucleic acid with a second forward primer comprising a target binding region, which binds to a complementary site in the adapter molecule at the 3' end of the target nucleic acid; conducting a sequencing-by-extension reaction wherein the target binding region of the second forward primer primes synthesis of a further complementary strand of the target nucleic acid and provides a sequencing read of the target nucleic acid; and displacing the further complementary strand from duplexing with the target nucleic acid. Optionally, the intervening region comprises an inverted nucleotide. Optionally, the intervening region comprises a non-nucleotide linkage. Optionally, the second target binding region is extension blocked and the method further comprises removing the block to extension before performing the extension primed by the second target binding region. Optionally, the second target binding region is extension blocked by a 3' terminal phosphate group and removing the block comprising treating with a phosphatase to remove the phosphate. Optionally, the second extension is performed with a strand-displacing polymerase. Optionally, the 3' adapter further comprises a sample index between the complementary sites for the first and second target binding regions.

In some methods, the 5' or 3' adapter or both further comprises a molecular index. In some methods, the 5' or 3' adapter or both further comprises a sample index. In some methods, the single-stranded nucleic acid flanked by the 5' and 3' adapters is produced by emulsion amplification. Some methods further comprise breaking the emulsion before performing other steps described above. In some methods, the support to which the single-stranded target nucleic acid is immobilized was used in emulsion PCR to generate the single-stranded target nucleic acid flanked by 5' and 3' adapters. In some methods, the support to which the single-stranded target nucleic acid is immobilized was attached after emulsion PCR. In some methods, the single-stranded target nucleic acid immobilized to a support is one of a clonal population of such target nucleic acids immobilized to the same support. In some methods, the support is a bead. In some methods, the support is an addressable region within an array. In some methods, the sequencing is by 454, ion torrent or ultima sequencing. In some methods , the sequencing is single molecule sequencing. In some methods, the sequencing is clonal sequencing. In some methods, the length of the target nucleic acid exceeds the maximum read length of the sequencing method. In some methods, the target nucleic acid is about 170 nucleotides and the maximum read length of the sequencing method is about 150 nucleotides of the target nucleic acid. In some methods, the sequencing reads have a non-overlapping region.

Some methods further comprises comprising determining a composite sequencing read from the forward and reverse sequencing reads in which any positions of discordance between forward and reverse sequencing reads are left open or as alternative nucleotides occupying the position in the forward and reverse sequencing reads.

The invention further provides a primer comprising first and second target binding regions each with a free 3' end separated by an inverted nucleotide.

The invention further provides a kit comprising a primer with first and second target binding regions, each with a free 3' end separated by an inverted nucleotide, first and second adapters, or a single adapter with asymmetric single-stranded regions disposed to flank individual strands of double-stranded target nucleic acid with first and second adapters, and a second primer, wherein the first and second target binding regions bind the first adapter and the second primer binds the second adapter. Optionally, one of the free 3' ends is blocked with a phosphate group and the kit further comprises a phosphatase to remove the phosphate.

The invention further provides a primer comprising a 3' target binding region and a 5' stem-loop, first and second adapters, or a single adapter with asymmetric single-stranded regions disposed to flank individual strands of double-stranded target nucleic acid with first and second adapters, a second primer and a third primer, wherein the 3' target binding region binds the first adapter, the second primer binds the loop region of the primer, and the third primer binds the second adapter. Any of the kits can also include a strand displacing polymerase.

### BRIEF DESCRIPTION OF THE FIGURES

Fig. 1 shows a clonal population of a single-stranded target nucleic acid flanked by different single-stranded adapters with the 5' adapter tethering the target nucleic acid to a support. The right portion of the figure shows a multifunctional primer with a 3' target binding region and a 5' stem loop region.
Fig. 2 shows the multifunctional primer of Fig. 1 annealed with the adapter at the 3' end of the target nucleic acid via its target binding region.
Fig. 3 shows extension primed by the target binding region of Fig. 2 with the target nucleic acid serving as a template, and ligation of the 5' end of the stem loop adapter to the 3' end of the 3' adapter attached to the target nucleic acid.
Fig. 4 shows extension of the target binding region referred to in Fig. 3 generating a forward sequencing read.
Fig. 5 shows the extension referred to in Fig. 4 having generated a complementary strand to the target nucleic acid (first complementary strand), and a loop primer annealing with the loop portion of the multifunctional primer.
Fig. 6 shows extension from the loop primer generating a second complementary strand and displacement of the original complementary strand referred to in Fig. 5, which remains linked to the target nucleic acid by the multifunctional primer and thus tethered to the support.
Fig. 7 shows a reverse sequencing primer annealing with the complement of the adapter at the 5' end of the target nucleic acid on the first complementary strand.
Fig. 8 shows extension from the reverse sequencing primer generating a reverse sequencing read.
Fig. 9 at left shows a multifunctional primer with a 3' target binding region and a 5' stem loop region as in Fig. 1 but here, the target binding region includes a uracil residue. At right the figure shows a multifunctional primer with a 3' target binding region and a 5' stem loop region blocked for extension by a phosphate group at the 3' end. Also shown is a linear primer annealing to the 3' adapter downstream of the multifunctional primer.
Fig. 10 shows a clonal population of a single-stranded target nucleic acid flanked by different single-stranded adapters with the 5' adapter tethering the target nucleic acid to a support. The 3' adapter includes two primer binding sites flanking an optional sample index.
Fig. 11 shows a multifunctional primer with two 3' target binding regions annealing with the 3' adapter shown in Fig. 10. The two 3' target binding regions are separated by an intervening region including an inverted base (dT).
Fig. 12 shows extension from the 3' target binding region proximate to the target nucleic acid generating a complementary strand (first complementary strand) and a forward sequencing read.
Fig. 13 shows unblocking of the 3' end of the 3' target binding region distal from the target nucleic acid in preparation for its extension.
Fig. 14 shows extension from the 3' target binding region distal from the target nucleic acid initially providing a sequencing read of the sample index.
Fig. 15 shows completed extension from the 3' target binding region distal from the target nucleic acid generating a further complementary strand and displacing the original complementary strand, which remains linked to the target nucleic acid by the multifunctional primer, and thereby tethered to the support.
Fig. 16 shows annealing of a reverse sequencing primer to the complement of the adapter at the 5' end of the target, which is now at the 3' end of the displaced original complementary strand. Extension of the reverse sequencing primer with the displaced original complementary strand serving as a template generates a reverse sequencing read.

### DEFINITIONS

A subject refers to an animal, such as a mammalian species (preferably human) or avian (e.g., bird) species, or other organism, such as a plant. More specifically, a subject can be a vertebrate, e.g., a mammal such as a mouse, a primate, a simian or a human. Animals include farm animals, sport animals, and pets. A subject can be a healthy individual, an individual that has symptoms or signs or is suspected of having a disease or a predisposition to the disease, or an individual that is in need of therapy or suspected of needing therapy.

A target nucleic acid refers to a nucleic acid whose sequence is to be at least partially determined. A target nucleic acid can be part of a population of nucleic acids, which may include other target nucleic acid and non-target nucleic acids present in the sample. The nucleic acids in a sample can be referred to collectively as sample nucleic acids. Target nucleic acids can be cell-free nucleic acids or derived from cell-free nucleic acid.

Sequencing can determine genetic variations. A genetic variation refers to a change in nucleotide sequence (nucleotide variation), modification, or copy number relative to that of a reference sequence, which can be e.g., an exon, gene, chromosome or full genome representing the normal sequence, modification, if any, and copy number for an organism. A genetic variation can include one or more single nucleotide variations (SNVs), insertions, deletions, repeats, small insertions, small deletions, small repeats, structural variant junctions, variable length tandem repeats, and/or flanking sequences, copy number variants (CNVs), transversions, gene fusions and other rearrangements, as well as modifications such as methylation, acetylation or hydroxymethylation are also forms of genetic variation. A variation can be a base change, insertion, deletion, repeat, copy number variation, modification, transversion, or any combination thereof.

A cancer marker is a genetic variation associated with presence or risk of developing a cancer. A cancer marker can provide an indication a subject has cancer or a higher risk of developing cancer than an age and gender matched subject of the same species that does not have the cancer marker. A cancer marker may or may not be causative of cancer.

The four standard nucleotide types refer to A, C, G, T for deoxyribonucleotides and A, C, T and U for ribonucleotides.

Within a sequencing read the terms "upstream" and "downstream" are used to indicate sequences relatively closer or further to the point of initiation of sequencing, typically a sequencing primer binding site. For example, if a sequencing read includes an upstream and downstream molecular barcode, the upstream molecular barcode is closer than the downstream molecular barcode to the point of initiation of sequencing.

A forward primer is a primer initiating first strand synthesis from an adapter or other template, and a reverse primer is a primer initiating second strand synthesis.

Unless otherwise apparent from the context, reference to a nucleic acid can include DNA, or RNA or hybrids thereof. Nucleic acid molecules isolated from nature typically contain standard nucleotides, including naturally modified forms thereof, such as methylcytosine. Synthetic oligonucleotides, such as adapters, can also be formed entirely from these standard nucleotides, or can include, one or more positions occupied by analogs of these standard nucleotides, capable of base pairing with one, some or all of the standard nucleotides. Nitroindole and deoxyinosine are examples of analog nucleotides capable of pairing with any of the standard nucleotides. Some synthetic oligonucleotides, such as adapters, are formed entirely of standard nucleotides of DNA. Some synthetic oligonucleotides, such as a adapters, include uracil or deoxyuridine as well as standard DNA nucleotides.

The term "support" refers to any solid or semi-solid article on which reagents such as nucleic acid molecules may be immobilized. Nucleic acid molecules may be synthesized, attached, ligated, annealed, or otherwise immobilized. Nucleic acid molecules can be immobilized on a substrate by any method including physical adsorption, by ionic or covalent bond formation, or combinations thereof. Supports can be planar, substantially planar, or nonplanar. A support can be two-dimensional (e.g., a planar 2D substrate) or three-dimensional. A support can be in the form of beads, spheres, particles, granules, a gel, or a porous matrix. A support can be a component of a flow cell and/or can be included within or adapted to be received by a sequencing instrument. A support can include a polymer, a glass, or a metallic material, organic polymers such as polystyrene, polyethylene, polypropylene, polyfluoroethylene, polyethyleneoxy, and polyacrylamide (e.g., polyacrylamide gel), as well as co-polymers and grafts thereof. A support can comprise latex or dextran. A support can be inorganic, such as glass, silica, gold, controlled-pore-glass (CPG), or reverse-phase silica. Examples of substrates include a membrane, a planar substrate, a microtiter plate, a bead (e.g., a magnetic bead), a filter, a test strip, and a slide. A support can be a single solid or semi-solid article (e.g., a single particle), or a plurality of such articles (e.g., a collection of particles). Substrates can be porous or non-porous, and can have swelling or non-swelling characteristics. A support can be shaped to comprise one or more wells, depressions, or other containers, vessels, features, or locations. A plurality of supports can be configured in an array at various locations. A support or features within a support can be addressable (e.g., for robotic delivery of reagents or detection, For example, a substrate can be in optical and/or physical communication with a detector or physically separated from a detector. A support n can be placed within or on another support (e.g., within a well of a second support) for sequencing reactions and detection.

A primer is a nucleic acid capable of annealing to a site within a nucleic acid template and priming extension of a complementary strand to the template. A primer includes a target binding region, which effects annealing to the template. The target binding region usually has a free 3' end. Exemplary lengths for the target binding region are at least 3, 5, 8, 9, 10, 11, 12, 13, 14, 15, 16, 17, 18, 19, or 20 nucleotides optionally up to 30, 35, 40, 45, 50, or more nucleotides including all permutations of upper and lower limits. The target binding region is perfectly complementary to a corresponding site on the template strand to which it anneals, or at least sufficient complementary to permit annealing and extension. A primer can also include a 5' segment non-complementary to the template strand, such as a hairpin loop that does not directly participate in annealing to the template or extension from the target binding region but provides an additional functionality. For example, the 5' segment can include a first sub-segment and a second sub-segment joined by an intervening segment, wherein the first and second sub-segments are complementary in opposing orientation, and wherein the intervening segment is capable of bending back on itself to allow the first and second sub-segments to base-pair with one another and form a double-stranded duplex. The first and second sub-segments of the 5' segment can be located at the ends of the 5' segment. The 5' segment of a stem-loop primer can be linked to the 3' segment either directly or via one or more intervening nucleobase units. Exemplary lengths for the 5' segments of stem-loop primers are at least 12, 15, 20, or 25 nucleobase units and optionally up to 35, 40, 45, 50, 55, 60 or more nucleobase units, including all permutations of upper and lower limits. A 5' segment of a primer can also include other functionalities, such as tag for labelling. Primers can be formed entirely of the four standard deoxyribonucleotides, or can include non-natural nucleotides or non-natural linkages between nucleotides, or non-nucleotide linkages as further described below.

A primer extension reaction refers to annealing of a primer to a template nucleic acid strand molecule, followed by elongation of the primer to generate a complementary strand to the template. Extension can incorporate nucleotides found in natural nucleic acids or analogs, sometimes labelled and/or chain terminating for purposes of sequencing. Extension may or may not be part of a sequencing by synthesis reaction. Sequencing-by-synthesis means an extension in which the identity of successive nucleotides added in an extension is detected to determine sequence. Priming and extension of one template strand may or may not be preceded by denaturation of a double-stranded template. Priming and extension of one template strand may or may not be followed by priming and extension of a complementary strand.

A polymerase is an enzyme capable of extending a primer annealed with a template by incorporation of nucleotides. Some examples of polymerases are T7 DNA polymerase, bacteriophage T4 DNA polymerase, PHI.29 (phi29) DNA polymerase, Taq polymerase, Tth polymerase, Tli polymerase, Pfu polymerase, Pwo polymerase, VENT polymerase, DEEPVENT polymerase, EX-Taq polymerase, LA-Taq polymerase, Sso polymerase, Poc polymerase, Pab polymerase, Mth polymerase, ES4 polymerase, Tru polymerase, Tac polymerase, Tne polymerase, Tma polymerase, Tea polymerase, Tih polymerase, Tfi polymerase, Platinum Taq polymerases, Tbr polymerase, Tfl polymerase, Pfutubo polymerase, Pyrobest polymerase, Pwo polymerase, KOD polymerase, Bst polymerase, Sac polymerase, Klenow fragment, polymerase with 3' to 5' exonuclease activity. Polymerases may or may not have strand displacement activity to displace a complementary strand already annealed with a template as a nascent strand is extended by the polymerase from a primer annealed with the template. T4 (NEB#M0203) and T7 (NEB #M0274) DNA Polymerases lack strand displacement activity, whereas phi29 (NEB #M0269) and SD polymerase (Ignatov, BioTechniques Volume 57, Issue 2, August 2014, Pages 81-87) have strong ability to strand displace.

The terms "duplex," "anneal" or "hybridization" and grammatic variations refer to sequence-specific pairing of nucleic acids, for example between a primer and template, with an affinity stronger than that between either component of such a pairing and other nucleic acids that may be present in a sample, and in the case of a primer and template sufficient for the primer to undergo templated-directed extension.

### DETAILED DESCRIPTION

### I. General

The invention provides methods of generating forward and reverse reads of an immobilized single-stranded target nucleic acid. The forward read can be obtained as a direct read of the single-stranded target from its 3' end to 5' end. The reverse read can be obtained from a complementary strand to the single-stranded target nucleic acid from the 3' end to 5' end of the complement. The provision of both forward and reverse sequence reads of the same target nucleic acid can be useful when the length of the target nucleic acid exceeds the read length of the sequencing technology. The forward and reverse reads can be combined after converting one such read to a sequences of complementary nucleotides to provide a total read length greater than either individual read. When independently synthesized, the presence of forward and reverse reads of the same target nucleic acid is also useful for error avoidance or correction as indicated by positions of discordance between the two reads. Such positions of discordance can be corrected or avoided in subsequent compilations of sequencing reads.

For example, if a sequencing technology has a maximum read length of 250 nucleotides of a target nucleic acid (not including additional read length used on flanking adapter sequences) and a target nucleic acid is 500 nucleotides length, then forward and reverse reads can in combination cover the entire length of the target nucleic acid. If a sequencing read length is 150 nucleotides of a target nucleic acid (again not including flanking adapter sequences) and the target nucleic acid is 170 nucleotides, then forward and reverse reads can cover the entire target nucleic acid as well as providing substantial overlap, which can be used to flag errors in the sequence read based on discordance.

The methods use multifunctional primers. Such a multifunctional primer serves to initiate separate syntheses of first and second complementary strands to a single-stranded target nucleic acid, and to tether the first complementary strand to immobilized target nucleic acid. The first complementary strand serves to provide a primer binding site and template for an extension product to generate a reverse sequencing read. The second complementary strand serves to displace the first strand from duplexing with the target nucleic acid. Either the first or second complementary strand can provide a forward sequence read.

The starting material for the methods is a single-stranded target nucleic acid flanked at its 5' end by a 5' adapter and at its 3'end by a 3' adapter. The adapters linked to the single-stranded target nucleic acid are single-stranded. The single-stranded target nucleic acid is immobilized through the 5' adapter to a support. The support can be, for example, a bead or the like or an addressable region of a planar array. The support can be the same support as used in synthesis or amplification of a target nucleic acid. Alternatively, the support used for synthesis or amplification, if any, can be removed before sequencing and a support attached to a target nucleic acid before sequencing. The adapters, or sometimes their complementary sequences for single-stranded adapters, provide primer binding sites. The 5' and 3' adapters have different primer binding sites, which can anneal or duplex with different primers. The starting target molecule flanked by adapters can be part of a clonal population of such molecules attached to the same support or same addressable region of a support or can be a single molecule.

The combination of any or all of a target nucleic acid flanked by adapters, one or more primers and reagents for extension and sequencing, such as a polymerase, nucleotides and label(s) is referred to as a reaction mix. The reagents can be mixed together initially or introduced in combinations or individually reflecting the order used as further described below.

The single-stranded target nucleic acid flanked by single-stranded adapters can be initially generated as a double-stranded target molecule flanked by double-stranded adapters. For example, a double-stranded target nucleic acid can be ligated to double-stranded adapters, or adapters can be linked to a target nucleic acid in the form of 5' tags to primers used in amplification. Before or after attaching adapters, target nucleic acids can be amplified. The adapters linked to a double-stranded target nucleic acid can be the same or different than each other. Ligation with a Y-shaped adapter or topological equivalents effectively allows the same initial adapter when ligated to a double-stranded target nucleic acid to generate component strands flanked by different single-stranded adapters. Adapters can also include molecular and/or sample bar codes. Double-stranded target nucleic acids flanked by double-stranded adapters can be converted to single-stranded form by denaturation, amplification or extension among other methods. Double-stranded or single-stranded target nucleic acids can be linked to a support. Linkage can be after synthesis and optionally denaturation of a double-stranded target nucleic acid or target nucleic acids can be synthesized nucleotide by nucleotide on a support or a planar array.

Emulsion amplification is one technique for providing a starting material suitable for the present methods. Emulsion amplification already forms a preliminary step in several sequences protocols including 454, Ion torrent and Ultima. Emulsion amplification provides a means of amplifying individual target molecules linked to adapters within separate compartments formed by an emulsion. Within each cell a clonal population of a target nucleic acid flanked by 5' and 3' adapters is formed immobilized to a bead. Different compartments in the emulsion contain beads with clonal populations of different target nucleic acids. After amplification, the emulsion can be broken mixing the beads and their attached clonal populations of target nucleic acid. Subsequent primer annealing and extension steps can be performed on the mixed beads in solution or after transferring the beads to a sequencing flow cell.

One method of the invention employs a forward primer comprising a 3' target binding region and a 5' stem loop, and a linear reverse primer. The 3' target binding region is configured to anneal to a 3' adapter flanking a target nucleic acid. The reverse primer is configured to anneal with a complement (i.e., complementary sequence ) of the 5' adapter. The length of complementary sequence in the primers required for such annealing is comparable to conventional primers (e.g., 10-25 contiguous nucleotides). Typically primers bind to complementary primer binding sites within an adapter but not occupying the full length of the adapter.

A target nucleic acid flanked by 5' and 3' adapters is contacted with a forward primer as described above under annealing conditions. For ease of illustration the method is described for a single target nucleic acid but can be performed in parallel with a population of target nucleic acids (e.g., at least 10, 100, 1000, or 1,000,000) usually flanked by the same 5' and 3' adapters (with the possible exception of molecular and/or sample barcodes). The forward primer anneals via its target binding region to the 3' adapter. The 3' end of the primer is then situated to prime extension of a first complementary strand to the target nucleic acid molecule. Before or after initiating such extension, the 5' end of the stem loop primer is ligated to the 3' end of the 3' adapter. The 3' end of the forward primer is extended with a polymerase with the target nucleic acid serving as a template and a first complementary strand to the target nucleic acid is synthesized. The extension can be a sequencing-by-synthesis reaction in which the identity of successive nucleotide types added to the forward primer are successively detected to generate a sequence of nucleotides constituting a forward sequencing read of the target nucleic acid. The extension can also be without sequencing, which can be referred to as a dark extension. If the extension is a dark extension the sequence can be determined separately by a non-sequencing by synthesis technology. The extension can be part sequencing-by-synthesis and part dark extension, in which case the part of the target nucleic acid corresponding to the dark extension can be sequenced separately. Alternatively, the sequence can be determined by conducting a separate sequencing-by-synthesis reaction of the target nucleic acid as further described below.

After completing synthesis of the first complementary strand whether by a sequencing-by-synthesis reaction and/ or dark sequencing a further extension is conducted primed by a loop primer which anneals with the loop region of the forward stem loop primer. This primer can be added to the reaction mix after completing the first extension or can have been added previously, in which case the 3' end can be blocked until extension from the loop primer is to be carried out. Blockage can be with a phosphate group and removal with a phosphatase. The loop primer anneals with the loop of the forward primer and primes extension with the target nucleic acid again serving as a template to generate a second complementary strand. This extension can be a dark extension, which serves to displace the previously synthesized complementary strand from the target nucleic acid. Alternatively, for example, if the previous extension to generate a complementary strand is a dark extension, the generation of the second complementary strand can be a sequencing-by-synthesis reaction to generate a forward sequencing read. This extension is preferably conducted with a strand displacing polymerase. Alternatively displacement can be effected by other means, such as denaturation. The displaced complementary strand remains covalently linked to the target nucleic acid by the forward stem loop primer.

The displaced first complementary stand has at its 3' end a complementary sequence to the 5' adapter. This complementary strand provides a binding site for a reverse primer. The reverse primer can be supplied after synthesis of the further complementary strand and displacement of the original complementary strand is complete or earlier in the process. The reverse primer after annealing to the complement of the 5' adapter can prime extension with the first complementary strand (i.e., generated from the 3' end of the stem loop primer) serving as a template. This extension can be a sequencing-by-synthesis step generating a sequence of nucleotides constituting a reverse sequence read. Alternatively it can be a dark extension and the sequence determined subsequently by a technology other than sequencing by synthesis.

The method described above generates the reverse sequencing read by reading a complementary strand to the target nucleic acid generated by the forward sequencing read. Thus, errors present in the forward sequencing read will also be present at corresponding positions of the reverse sequencing read. The method can be modified to generate independent forward and reverse sequencing reads. One such modification is to use a second forward primer, usually before use of the stem loop forward primer. The second primer is a typically a linear primer having a target binding region that duplexes at a site within the 3' adapter, the same or different than that duplexed by the stem loop forward primer. The second forward primer can prime synthesis of a complementary strand with the target nucleic acid as a template generating a sequencing read of the target nucleic acid. This complementary strand is then displaced by denaturation or digestion or use of a strand displacing polymerase for the next extension. The method then proceeds as previously described using a stem loop forward primer to prime synthesis of a complementary strand to the target nucleic acid. But if a forward read of the target nucleic acid has already been determined, this extension can be a dark extension. Ligation of the hairpin, annealing of a loop primer, extension of another complementary strand, strand displacement annealing of a reverse primer and extension to determine a reverse strand sequencing read then proceed as previously described.

A further variation to obtain independent forward and reverse sequencing reads uses the stem loop forward primer, loop primer and reverse primer as previously described. However, the initial extension of the stem loop primer to generate a first complementary strand is a dark extension. The subsequent extension from the loop primer annealed with the stem loop primer of a second complementary strand of the target nucleic acid as well as displacing the first complementary strand is now a sequencing-by-synthesis reaction that provides a forward sequencing read.

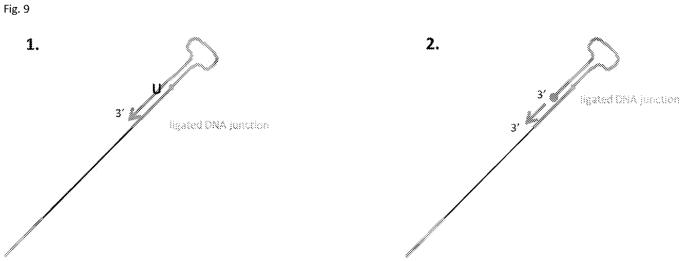

Another variation uses the stem-loop forward primer, loop primer and reverse primer as previously described, but the stem-loop forward primer now contains a cleavable nucleotide within its target binding region (Fig. 9 left). An example of a cleavable nucleotide is uracil or deoxyuridine, which can be cleaved by RNase H digestion. An extension primed from the 3' end of the stem loop forward primer is carried out as previously described to generate a complementary strand to the target nucleic acid and a forward sequencing read. The cleavable nucleotide is now cleaved, and the complementary strand just synthesized and a stub of the stem loop forward primer left after cleavage is then dissociated from the target nucleic acid. Another extension is then conducted from a newly generated 3' end of the forward stem loop primer. This extension generates another complementary strand of the target nucleic acid. If conducted with a strand displacing polymerase, this extension can cause displacement of the complementary end generated prior to cleavage. The extension to generate the further complementary strand can be a dark extension because the forward sequencing read has already been determined. Thereafter, the method proceeds as originally described with extension from the loop primer annealed with the loop region of the forward stem loop primer to generate another complementary strand displacing the previously synthesized complementary strand, which provides a primer binding site for the reverse primer. The reverse primer primes synthesis of the complement of the displaced complementary strand allowing determination of a reverse sequencing read.

In another variation, the forward stem loop primer is used in conjunction with a separate linear forward primer (Fig. 9 right). The linear forward primer and the target binding region of the forward stem loop primer anneal to adjacent or proximate site, such that the forward stem loop primer is between the linear primer and the 3' end of the 3' adapter. Together the linear forward primer and the forward stem loop primer can be viewed as being a split primer topologically analogous to the previously described forward stem loop primer with a cleavable site after cleavage has occurred. The forward linear primer primes extension of a first complementary strand with the target nucleic acid serving as template. This extension can be a sequencing-by-synthesis extension to provide a forward sequencing read. Extension from the forward stem loop primer then proceeds analogously to extension from the forward stem loop primer end generated by cleavage as previously described. The remaining steps are also the same as for the forward stem loop primer with a cleavable nucleotide.

An alternative method for determining forward and reverse sequencing reads of a single-stranded target nucleic acid flanked by 5' and 3' adapters as described above and immobilized via the 5' adapter, uses a forward primer comprising first and second 3' target binding regions flanking an intervening region in place of the forward stem-loop primer. The intervening region contains a non-standard nucleotide linkage or non-nucleotide linkage to permit j oinder of two 3' target binding regions in the same primer. An inverted nucleotide provides an example of a suitable linkage. The first and second target binding regions bind complementary sites in the 3' adapter molecule, the respective sites being distal and proximal to the 3' end of the 3' adapter. In other words, when annealed the second target binding region is between the first target binding region and the 3' end of the 3' adapter. The two 3' target binding regions provide two separate points to initiate extension of a first complementary strand from within the 3' adapter. Extension from the first target binding site is analogous to extension from the 3' end of the forward stem loop primer in the previously described method and extension from the second target binding site is analogous to extension from the loop primer annealed with the forward stem loop primer in the previously described method. The second target binding region can be extension blocked, such as by presence of a phosphate group, to prevent it priming extension until extension from the first target binding region has been completed. Extension from the first target binding region generates a first complementary strand to the target nucleic acid. The extension can be a sequencing-by-synthesis reaction to generate a forward sequencing read or a dark extension. If a dark extension, the sequence can be determined by a sequencing method other than sequencing by synthesis or the forward sequencing read can be determined from a different extension as will be further described. The extension can also be part sequencing-by-synthesis and part dark extension. After generation of the first complementary strand from the first target binding synthesis, the second target binding site is unblocked if necessary, and used to primer extension of a second complementary strand with the target nucleic acid serving as a template. This extension can be a sequencing-by-synthesis extension to generate a forward sequencing read or a dark extension. The extension is preferably conducted with a strand-displacing polymerase to displace the originally synthesized complementary strand. Displacement can alternatively be the result of denaturation. After displacement, the first complementary strand remains covalently linked to the forward primer, which is also covalently linked to the second complementary strand, which is in turn duplexed with the target nucleic acid molecule, which is immobilized via its 5 adapter. Thus, the displaced first complementary strand remains tethered to the immobilized target nucleic acid. A reverse primer is supplied which anneals with a complementary sequence of the 5' adapter present at the 3' end of the displaced first complementary strand. The annealed primer can then prime synthesis of a strand complementary to the displaced first complementary strand. This extension can be a sequencing-by-synthesis extension or the sequence can be determined separately after the extension is complete. In either case, a reverse sequence read is generated.

As was described for the stem-loop primer method, the above-described method can be modified to generate independent forward and reverse sequencing reads. For example, an initial extension can be performed with a second forward primer, typically a linear primer, complementary to a site in the 3' adapter. This extension can be a sequencing-by-synthesis reaction to generate a forward sequencing read or sequencing can be performed after extension to generate a forward sequencing read. After this extension, the forward primer with first and second 3' target binding regions is annealed. The first target binding site is used to prime synthesis of a complementary strand to the target nucleic acid, which displaces the complementary strand extended from the second forward primer. This extension is typically a dark extension. The second target binding site is used to primer synthesis of another complementary strand to displace the complementary strand extended from the first 3' target binding region. This extension is also typically a dark extension. A reverse primer is annealed to the complement of the 5' adapter now at the 3' end of the displaced complementary strand and reverse strand sequencing proceeds are previously described.

Practice of the above methods generates a forward sequencing read and a reverse sequence read of one or more target nucleic acid. Nucleotide types in reverse sequencing reads can be converted to their complements for consistency with the forward sequencing read or vice versa. Sequencing reads can be aligned and compared pairwise, with sequencing reads of another target nucleic acid or with a known reference sequence. Pairwise alignment of forward and reverse sequence reads can generate a composite sequencing read longer than either the forward or reverse sequencing read. At positions in pairwise alignment where there is discordance between forward and reverse sequencing reads, the discordance can be indicated by not assigning a nucleotide at that position in the composite sequence (i.e., leaving it open) or assigning a position of ambiguity where either of the nucleotides present in the forward and reverse sequence reads can be present. The omitted position or ambiguous position can be resolved by alignment with sequencing reads of other target nucleic acids of at least overlapping sequence where the same position is not subject to the same omission or ambiguity. Individual forward and reverse sequencing reads or composite sequencing reads from pairwise alignment of forward and reverse sequencing reads of the same target nucleic acid can be grouped into families according to sample molecule of origin and further analyzed as described below.

### II. Sample and molecular barcodes and adapters

As previously mentioned, adapters can include sample and/or molecular barcodes. A barcode is a short nucleic acid (e.g., less than 500, 100, 50, 20, 15, 10 or 5 nucleotides long), used to label nucleic acid molecules to distinguish nucleic acids from different samples (a sample barcode), or different nucleic acid molecules in the same sample (a molecular barcode) or the same barcode can be used to distinguish both samples and molecules within samples.

Barcodes are typically provided as sets of multiple different individual barcodes for distinguishing samples and molecules or both. That is, different samples can receive different sample barcodes from a set of sample barcodes, and different molecules within a sample receive different molecular barcodes from a set of molecular barcodes. Barcodes can be single-stranded, double-stranded or have both single and double-stranded components. Barcodes can have the same or different lengths within a set. Barcodes can be random, non-random or semi-random sequences in which at least one position is randomly selected and at least one is not. Barcodes can be synthesized together with pooling of nucleotides at random positions, or individually. Some sets of barcodes having sequences selected such that there is a Hamming distance of at least 2, 3, 4 or 5 nucleotides between each barcode in a set. Barcodes can also be selected to avoid sequences that hybridize within one another or other molecules within a reaction, to avoid sequences subject to sequencing errors, or sequences subject to confusion with sequences of other barcodes. Barcodes as components of adapters or tails of amplification primers can be attached to one end or both ends of nucleic acids to be labelled.

Sample barcodes can be decoded to reveal sample of origin. Sample barcodes allowing pooling and parallel processing of multiple samples after the barcodes have been attached. The number of a different sample barcodes within a set is typically sufficient that each different sample is associated with a different sample barcode or combination of barcodes. Alternatively, samples can be divided into subsets with samples in a subset receiving the same sample barcode and samples in different subsets receiving different sample barcodes.

Molecular barcodes are used to track original molecules within the same sample. They can be decoded to reveal amplification copies or sequencing reads thereof of the same original molecule. The number of molecular barcodes within a set or number of pairwise combinations within a set if sample molecules are labelled with molecular barcodes from both ends can be sufficient such that there is a high probability (e.g., at least 80, 90, 95 or 99% probability) that substantially all original molecules in sample that complete ligation with an adapter or pair of adapters (e.g., at least 75%, 90%, 95% or 99%) receives a different molecular barcode or different combination of molecular barcodes (unique barcoding). Alternatively, the number of molecular barcodes or pairwise combinations of molecular barcodes can be substantially less than the number of molecules within a sample, e.g., a ratio of different molecular barcodes or pairwise combination of molecular barcodes to samples molecules of less than 1:10³, 1:10⁴, 1:10⁵, 1-10⁶, 1:10⁷, 1-10⁸ ,1:10⁹, 1: 10^{10,} 1:10¹¹ or 1:10¹² non-unique barcoding). In this case, multiples molecules within the same sample receive the same molecular barcode or combination of molecular barcodes. However, amplification products of the same original molecule or their sequencing reads can still be distinguished by using a combination of the molecular barcodes and information from the sequencing reads, such as the start and/or stop points or length of sequencing reads. Typically sufficient different molecular barcodes or combinations of molecular barcodes are used such that there is high probability (e.g., at least 90%, at least 95%, at least 98%, at least 99%, at least 99.9% or at least 99.99%) that all nucleic acids mapping to a particular genomic region defined by same start and/or stop points bear a different molecular barcode. Generally, assignment of unique or non-unique molecular barcodes in reactions follows methods and systems described by US patent applications 20010053519, 20030152490, 20110160078, and U.S. Pat. No. 6,582,908 and U.S. Pat. No. 7,537,898.

In some cases, the number of different molecular barcodes is at least 2, 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000. In other cases, the number of different molecular barcodes is less than 3, 4, 5, 6, 7, 8, 9, 10, 20, 50, 100, 500, 1000, 5000, 10000, 50,000, 100,000, 500,000, 1,000,000, 10,000,000, 50,000,000 or 1,000,000,000 molecular barcodes per genome sample. The number of different molecular barcodes in a set depends on whether unique or nonunique barcoding is used and whether molecular barcodes are used to label nucleic acid sample molecules individually or in pairwise combinations. Other things being equal, more different molecular barcodes are needed for unique than non-unique labelling. Also more different molecular barcodes are needed for labelling with individual molecular barcodes per sample nucleic acid than in pairwise combinations, because the number of combinations is the square of the number of individual labels.

The number of different molecular barcodes necessary for unique labelling of nucleic molecules is a function of how many original nucleic acid molecules are in the sample or part thereof being analyzed. This, in turn, depends on such factors at the total number of haploid genome equivalents in the sample, the average and variance in size of nucleic acid molecules, and the ligation efficiency of adapters including barcodes.

For non-unique barcoding the number of molecular barcode combinations (square of number of different molecular barcodes) is sometimes least any of 64, 100, 400, 900, 1400, 2500, 5625, 10,000, 14,400, 22,500 or 40,000 and no more than any of 90,000, 40,000, 22,500, 14,400 or 10,000. For example, the number of barcode combinations can be between 64 and 90,000, between 400 and 22,500, 400 and 14,400 or between 900 and 14,400. The number of different molecular barcode combinations (n) can be between 2 and 100,000*z, wherein z is a measure of central tendency (e.g., mean, median, mode) of an expected number of duplicate molecules having the same start and stop positions. The number of different molecular barcode combinations can be at least any of 2*z, 3*z, 4*z, 5*z, 6*z, 7*z, 8*z, 9*z, 10*z, 11*z, 12*z, 13*z, 14*z, 15*z, 16*z, 17*z, 18*z, 19*z, 20*z or 100*z (e.g., lower limit). Optionally, n is no greater than 100,000*z, 10,000*z, 2000*z, 1000*z, 500*z or 100*z (e.g., upper limit). Thus, n can range between any combination of these lower and upper limits. The number of combinations can be between 100*z and 1000*z, 5*z and 15*z, between 8*z and 12*z, or about 10*z. For example, a haploid human genome equivalent has about 3 picograms of DNA. A sample of about 1 microgram of DNA contains about 300,000 haploid human genome equivalents. The number n can be between 15 and 45, between 24 and 36, between 64 and 2500, between 625 and 31,000, or about 900 and 4000. For example, a sample comprising about 10,000 haploid human genome equivalents of cfDNA can be barcoded with about 36 combinations of six different molecular barcodes. Samples barcoded in such a way can be those with a range of about 10 ng to any of about 100 ng, about 1 µg, about 10 µg of fragmented polynucleotides, e.g., genomic DNA, e.g. cfDNA.

Adapters are relatively short nucleic acids for attachment to the ends of sample molecules to facilitate immobilization, amplification, sequencing and tracking of target nucleic acids. Adapters can be double-stranded with the possible exception of cohesive ends to facilitate ligation. Adapters can also be single-stranded. Adapters can also have a double-stranded portion and a single-stranded portion as in Y-shaped adapters and topological equivalents. The total length of each adaptor (measured by the longest strand if more than one) is e.g., less than 250, 150, 100, 75 or 50 nucleotides long. Adapters can include primer binding sites to permit binding of amplification, extension and/or sequencing primers. Adapters can also include binding sites for capture probes, such as an oligonucleotide attached to a flow cell support or a bead.

Some adapters have one or more double-stranded portions and one or more single-stranded portions. Y-shaped adapters (see, e.g., US 7,741,463), stem-loop (see e.g., US 10,155,939) and bubble adapters (see US20180030532A1) are examples of such adapters. Y-shaped adapters are nucleic acids formed from two strands, which are paired in a double-stranded portion (with the possible exception of a single-stranded overhang to facilitate ligation), and also unpaired in single-stranded portions. The two single-stranded portions can be represented in the shape of the letter V joined to the double-stranded portion, together forming a Y-shape. Y-shaped adapters have one free end in the double-stranded portion, which can be a blunt end or an end in which one strand overhangs the other, e.g., by a single nucleotide. Each of the unpaired single strands has a single-stranded end. The total length of each strand of Y-shaped adapters is e.g., less than 250, 150, 100, 75 or 50 nucleotides long. A standard Illumina Y-shaped adapter without sample or molecular barcodes has a strand length of about 115 nucleotides. The free end of the double-stranded portion serves for joining of a sample molecule (e.g., by blunt or cohesive end ligation).

Stem-loop adapters (e.g., NebNext from New England Biolabs) are similar to Y-shaped adapters except that the single-stranded portions are joined via a uracil residue thus forming a loop instead of a V. Thus, stem-loop adapters are a single strand with a duplexed stem corresponding to the double-stranded portion of Y-shaped adapters, and a loop including two single-stranded portions of DNA separated by a uracil (U) or deoxyuridine (dU), which correspond to the single-stranded portions of Y-shaped adapters. The residues immediately adjacent the U or dU are the single-stranded-end residues of the single-stranded portions in stem-loop adapters. The stem has a free end that can be blunt or tailed as in the stem of Y-shaped adapters and is used for joining to a sample molecule. After joining of stem-loop adapters to a sample molecule, the U or dU can be enzymatically removed leaving the same topography as for Y-shaped adapters. USER Enzyme from NEB is a mixture of Uracil DNA glycosylase (UDG) and the DNA glycosylase-lyase Endonuclease VIII (DGLE). UDG catalyzes the excision of a uracil or deoxyuridine base, forming an abasic (apyrimidinic) site while leaving the phosphodiester backbone intact, and DGLE removes the abasic nucleotide.

Bubble adapters (BGI) are similar to stem-loop adapters and Y-shaped adapters except that the V-region of Y-shaped adapter or the loop of stem-loop adapters is replaced by a bubble of two unduplexed single-stranded portions flanked on both sides by double-stranded portions. Bubble adapters typically have two strands of unequal length with some or all of the length difference being in the single-stranded portions. The 5' end of the longer nucleic acid has a phosphorylated nucleotide. The 3' end of the shorter nucleic acid typically has an overhang from the end of an otherwise double-stranded portion. The double-stranded portion containing the phosphorylated 5' nucleotide and overhang if present corresponds with the stem of stem-loop adapters or the double-stranded portion of Y-shaped adapters, and ligates with a sample nucleic acid molecule. This double-stranded portion can be referred to as the downstream double-stranded portion because it provides the site of ligation to a sample molecule. The other double-stranded portion can be referred to an upstream double-stranded portion because it is further from the sample molecule. The two single-strands in the middle forming a bubble correspond with the single-stranded portions forming a V in Y-shaped adapters or the single-stranded portions separated by a uracil or deoxyuridine in stem-loop adapters. Bubble adapters can include a U or dU in the shorter strand, longer strand or both to separate the single-stranded portions from the upstream double-stranded portion. Usually such a U or dU is included in the longer strand. The U or dU can be excised as with stem-loop adapters after ligation of the adapters to sample molecules leaving adapters in a Y-shape.

Primer binding sites are typically provided in the single-stranded portions of a Y-shaped , stem-loop or bubble adapter. The asymmetry of unpaired single- stranded portions allows strand-specific sequencing from two primers binding to the respective single strands. Adapters can also include binding sites for capture probes, such as an oligonucleotide attached to a flow cell support.

The same (with the possible exception of barcodes) or different adapters can be linked to the respective ends of a nucleic acid molecule. The sequences of adapters and particularly the segments for primer binding attachment to a flow cell can vary depending on the sequencing platform employed.

### III. Preparation of target nucleic acids

The methods provide forward and reverse sequence reads of at least one target nucleic acid and often of populations of at least 100, 1000, 10,000, 100,000 or 1 M different target nucleic acids. Target nucleic acid(s) can be obtained as some or all of a population of nucleic acids in sample from a subject or plurality of samples from a plurality of subject or the same subject at different times or from different sources (i.e., tissues or fluids) in the same subject.

A sample can be any biological sample isolated from a subject. Samples can include body tissues, such as known or suspected solid tumors, whole blood, platelets, serum, plasma, stool, red blood cells, white blood cells or leucocytes, endothelial cells, tissue biopsies, cerebrospinal fluid synovial fluid, lymphatic fluid, ascites fluid, interstitial or extracellular fluid, the fluid in spaces between cells, including gingival crevicular fluid, bone marrow, pleural effusions, cerebrospinal fluid, saliva, mucous, sputum, semen, sweat, urine. Samples are preferably body fluids, particularly blood and fractions thereof, and urine. A sample can be in the form originally isolated from a subject or can have been subjected to further processing to remove or add components, such as cells, or enrich for one component relative to another. Thus, a preferred body fluid for analysis is plasma or serum containing cell-free nucleic acids.

Use of sample barcodes permits pooling of samples with subsequent deconvolution from the barcodes. The number of different samples can be greater than or equal to 2, 5, 10, 50, 100, 500, 1000, 2000, 5000, or 10,000. The volume of plasma can depend on the desired read depth for sequenced regions. Exemplary volumes are 0.4-40 mL, 5-20 mL, 10-20 mL. For examples, the volume can be 0.5 mL, 1 mL, 5 mL 10 mL, 20 mL, 30 mL, or 40 mL. A volume of sampled plasma may be for example 5 to 20 mL.

A sample can comprise various amount of nucleic acid that contains genome equivalents. For example, a sample of about 30 ng DNA can contain about 10,000 haploid human genome equivalents and, in the case of cell-free DNA, about 200 billion individual nucleic acid molecules. Similarly, a sample of about 100 ng of DNA can contain about 30,000 haploid human genome equivalents and, in the case of cell-free DNA, about 600 billion individual molecules. Some samples contain 1-500, 2-100, 5-150 ng cell-free DNA, e.g., 5-30 ng, or 10-150 ng cell-free DNA.

cfDNA has a peak of fragments at about 160 nucleotides, and most of the fragments in this peak range from about 140 nucleotides to 180 nucleotides. Accordingly, cfDNA from a genome of about 3 billion bases (e.g., the human genome) may be comprised of almost 20 million (2x10⁷) polynucleotide fragments. A sample of about 30 ng DNA can contain about 10,000 haploid human genome equivalents. (Similarly, a sample of about 100 ng of DNA can contain about 30,000 haploid human genome equivalents.) A sample containing about 10,000 (10⁴) haploid genome equivalents of such DNA can have about 200 billion (2x10¹¹) individual polynucleotide molecules. It has been empirically determined that in a sample of about 10,000 haploid genome equivalents of human DNA, there are about 3 duplicate polynucleotides beginning at any given position. Thus, such a collection can contain a diversity of about 6x10¹⁰-8x10¹⁰ (about 60 billion-80 billion e.g., about 70 billion (7x10¹⁰)) differently sequenced polynucleotide molecules.

A sample can comprise nucleic acids of different types and origins. A sample can contain DNA or RNA or both. Nucleic acids can be single-stranded or double-stranded or be partly double-stranded and partly single-stranded. A sample can comprise germline DNA or somatic DNA or both. Nucleic acids within a sample can carry genetic variations, which can be carrying germline mutations and/or somatic mutations. Some such mutations can be cancer markers (e.g., cancer-associated somatic mutations).

Exemplary amounts of cell-free nucleic acids in a sample before amplification range from about 1 fg to about 1 ug, e.g., 1 pg to 200 ng, 1 ng to 100 ng, 10 ng to 1000 ng. For example, the amount can be up to about 600 ng, up to about 500 ng, up to about 400 ng, up to about 300 ng, up to about 200 ng, up to about 100 ng, up to about 50 ng, or up to about 20 ng of cell-free nucleic acid molecules. The amount can be at least 1 fg, at least 10 fg, at least 100 fg, at least 1 pg, at least 10 pg, at least 100 pg, at least 1 ng, at least 10 ng, at least 100 ng, at least 150 ng, or at least 200 ng of cell-free nucleic acid molecules. The amount can be up to 1 femtogram (fg), 10 fg, 100 fg, 1 picogram (pg), 10 pg, 100 pg, 1 ng, 10 ng, 100 ng, 150 ng, or 200 ng of cell-free nucleic acid molecules. The method can comprise obtaining 1 femtogram (fg) to 200 ng.

An exemplary sample is 5-10 ml of whole blood, plasma or serum, which includes about 30 ng of DNA or about 10,000 haploid genome equivalents.

Some samples contain cell-free nucleic acids. Cell-free nucleic acids are nucleic acids not contained within or otherwise bound to a cell or in other words nucleic acids remaining in a sample of removing intact cells. Cell-free nucleic acids include DNA, RNA, and hybrids thereof, including genomic DNA, mitochondrial DNA, siRNA, miRNA, circulating RNA (cRNA), tRNA, rRNA, small nucleolar RNA (snoRNA), Piwi-interacting RNA (piRNA), long non-coding RNA (long ncRNA), or fragments of any of these. Cell-free nucleic acids can be double-stranded, single-stranded, or a hybrid thereof. Double-stranded DNA molecules at least some of which have single-stranded overhangs are a preferred form of cell-free DNA for any method disclosed herein. A cell-free nucleic acid can be released into bodily fluid through secretion or cell death processes, e.g., cellular necrosis and apoptosis. Some cell-free nucleic acids are released into bodily fluid from cancer cells e.g., circulating tumor DNA, (ctDNA). Others are released from healthy cells.

A cell-free nucleic acid can have one or more epigenetic modifications, for example, a cell-free nucleic acid can be acetylated, methylated, ubiquitinylated, phosphorylated, sumoylated, ribosylated, and/or citrullinated.

Cell-free nucleic acids have a size distribution of about 100-500 nucleotides, particularly 110 to about 230 nucleotides, with a mode of about 168 nucleotides and a second minor peak in a range between 240 to 440 nucleotides.

Cell-free nucleic acids can be isolated from bodily fluids through a partitioning step in which cell-free nucleic acids, as found in solution, are separated from intact cells and other non-soluble components of the bodily fluid. Partitioning may include techniques such as centrifugation or filtration. Alternatively, cells in bodily fluids can be lysed and cell-free and cellular nucleic acids processed together. Generally, after addition of buffers and wash steps, nucleic acids can be precipitated with an alcohol. Further clean up steps may be used such as silica based columns to remove contaminants or salts. Non-specific bulk carrier nucleic acids, for example, may be added throughout the reaction to optimize certain aspects of the procedure such as yield.

After such processing, samples can include various forms of nucleic acid including double-stranded DNA, single-stranded DNA and single-stranded RNA. Optionally, single-stranded DNA and RNA can be converted to double-stranded forms so they are included in subsequent processing and analysis steps.

Nucleic acids present in a sample with or without prior processing as described above typically contain a substantial portion of molecules in the form of partially double-stranded molecules with single-stranded overhangs. Such molecules can be converted to blunt-ended double-stranded molecules by treating with one or more enzymes to provide a 5'-3' polymerase and a 3'-5' exonuclease (or proof reading function), in the presence of all four standard nucleotide types. Such a combination of activities can extend strands with a recessed 3' end so they end flush with the 5' end of the opposing strand (in other words generating a blunt end) or can digest strands with 3' overhangs so they are likewise flush with the 5' end of the opposing strand. Both activities can optionally be conferred by a single polymerase. The polymerase is preferably heat-sensitive so that its activity can be terminated when the temperature is raised. Klenow large fragment and T4 polymerase are examples of suitable polymerase.

The resulting blunt-ended nucleic acids can be ligated to adapters with a double-stranded blunt free end or can be subject to tailing to generate cohesive ends, which pair with corresponding single-stranded overhangs at a double-stranded free end of adapters. Tailing of blunt ends can be by a polymerase lacking a proof reading function. This polymerase is preferably thermostable such as to remain active at the elevated temperature that denatures the polymerase use for blunt ending. Taq, Bst large fragment and Tth polymerases are examples of such a polymerase. The second polymerase effects a non-templated addition of a single nucleotide to the 3' ends of blunt-ended nucleic acids. Although the reaction mixture typically contains equal molar amounts of each of the four standard nucleotide types from the prior step, the four nucleotide types are not added to the 3' ends in equal proportions. Rather A is added most frequently, followed by G followed by C and T. Such tailed molecules can be ligated to adapters with a complementary T or C overhand at the free end of the double-stranded portion.

For samples containing initially double-stranded nucleic acids, the present methods preferably result in at least 75, 80, 85, 90 or 95% of double-stranded nucleic acids in the sample being linked to adapters. The resulting nucleic acid are denatured to single-stranded form to perform the present methods. Preferably, the present methods result in at least 75, 80, 85, 90 or 95% of initially double-stranded molecules in the sample being sequenced.

### IV. Amplification

Sample nucleic acids flanked by adapters can be amplified by PCR and other amplification methods typically primed from primers binding to primer binding sites in adapters flanking a nucleic acid to be amplified. Amplification methods can involve cycles of extension, denaturation and annealing resulting from thermocycling or can be isothermal as in transcription mediated amplification. Other amplification methods include the ligase chain reaction, strand displacement amplification, nucleic acid sequence based amplification, and self-sustained sequence based replication. Amplification can be performed once or multiple times. Amplification can be performed before and distinct from sequencing or integrated with sequencing or both. Amplification can also be performed before or after enrichment of selected sample molecules, or both.

Emulsion amplification is a commonly employed method for amplification of nucleic acids in multiple next generation sequencing platforms (see, e.g., Kanagal-Shamanna Methods Mol Biol. 2016; 1392:33-42. doi: 10.1007/978-1-4939-3360-0_4. PMID: 26843044). The basic principle of emulsion amplification is dilution and compartmentalization of nucleic acids to be amplified in water droplets in a water-in-oil emulsion. Preferably the dilution is to a degree where each droplet contains a single target nucleic acid and functions as a micro-reactor. The compartmentalization of individual nucleic acid in distinct reaction droplets allows their amplification independently of one another, thereby reducing formation of chimeric by-products and overall amplification bias. The nucleic acids subject to amplification can be the result of prior fragments of larger molecules, such as by digestion of a sample of genomic DNA. Nucleic acids can be attached to adapters before amplification and amplification can provide sites for primer binding. Nucleic acids, typically flanked by adapters, are immobilized to supports, before, and/or during amplification. Supports are usually substantially spherical beads, or other particles, sized so that a single droplet can accommodate a support. Amplification requires primer(s), salts, a polymerase and nucleotides triphosphates as in other forms of amplification. The amplification is configured such that a clonal population of amplicons of the same original nucleic acid molecule becomes attached to the same support within a compartment. Such a configuration can be achieved by linking multiple copies of a forward primer to each support. The forward primer is complementary to an adapter at the 5' end of a nucleic acid strand to be amplified. This strand and copies of it generated during amplification can anneal to the forward primer of the support. Extension of the primer generates complementary strand covalently linked to the primer, which is in turn attached to the support. The end result is a clonal population of single-stranded nucleic acid joined via a 5' adapter to a support. Different supports are attached to different clonal populations. After amplification, the emulsion can be broken mixing the supports. Further extension and/or sequencing reactions can be performed on the resulting population of mixed supports, or the supports can be redistributed to individual wells or the like of sequencing flow cells first.

### V. Enrichment

Sample nucleic acid populations can be subject to enrichment for target nucleic acids of interest. Enrichment can be performed by affinity purification, e.g., by hybridization to immobilized oligonucleotides complementary to the sequences of interest. Enrichment can be performed before or after ligation to adapters, and before or after amplification, or any combination thereof.

### VI. Sequencing

The term "sequencing" refers to a process for generating a sequence of a target nucleic acid. Sequencing can be performed on individual molecules (single molecule sequencing) or clonal populations originating from the same molecule (clonal sequencing). Sequencing can be part of a template-directed primer extension (sequencing by synthesis) in which successive nucleotides incorporated into a nascent chain are detected. Sequencing can also be independent of extension, such as in sequencing by hybridization or ligation. Sequence generated from a single template or clonal population of the same templates can be referred to as a sequencing read. A sequencing read can include sequence of part or all of a target nucleic acid as well as some or all of adapters flanking the target nucleic acid. For example, a sequencing read can include at least 5, 10, 15, 20, 50, 100, 250, or 500 nucleotides of sequence from a target nucleic acid. The nucleotides in a sequence read can correspond to contiguous nucleotides in a target nucleic acid or can have contiguous stretches separated by gap(s) of one or more positions where the identity of a nucleotide is not determined. Any gaps preferably occupy no more than 1%, 5% or 10% of positions in a sequencing read. A sequencing read can be formed of the nucleotide types present in a single-stranded target nucleic acids or alternatively formed of their complementary nucleotides types. A forward and reverse sequencing reads refers to sequence generated in opposing directions with respect to a single-stranded target nucleic acid. The forward sequencing read is determined 3'-5' with respect to a single-stranded template and the reverse sequencing read 3'-5' with respect to the complement of the template or 5'-3 with respect to the template itself.

454 pyrosequencing is one example of a sequencing method (Siqueira et al., J Oral Microbiol. 2012;4:10.3402/jom.v4i0.10743. doi:10.3402/jom.v4i0.10743). Individual nucleic acids to be sequenced are ligated to adapters and tethered to individual beads, preferably one original fragment per bead. Nucleic acids are then amplified on the beads such that a clonal population forms on the beads. Amplification is followed by chemical detection of DNA synthesis reactions primed by the amplicons in a miniature chamber where pyrophosphate release is measured. By consecutively flooding such a chamber with sequencing reagents containing one of the 4 standard nucleotides, when the correct nucleotide is incorporated in the synthesized strand, pyrophosphate release is measured utilizing a light-generating reaction. The intensity of light also provides information concerning homopolymer runs of nucleotides in the sequence. Pyrosequencing was developed by Pyrosequencing AB, and subsequently acquired by Qiagen who licensed it to 454 Life Sciences, before it was ultimately acquired by Roche.

Ion Torrent is another example of a sequencing method (see Hu et al., Human Immunology 82, 801-811 (2021)). Nucleic acids to be sequenced are attached to adapters. The adapted DNA fragments are then attached to beads, preferably one bead for one fragment. Fragments are then amplified on the beads by emulsion amplification to generate large populations of beads each having a clonal population of the same original molecule. The beads are then flowed across the chip containing the wells configured such that only one bead can enter an individual well. When the sequencing reagents are then flowed across the wells, when the appropriate nucleotide is incorporated, a hydrogen ion is given off and the signal recorded. Nucleotide incorporation is directly converted to voltage which is recorded directly. The Ion Torrent system is sold by Thermo-Fisher and several versions of the platform are available, including Ion Personal Genome Machine^{™} (PGM^{™}) System, Ion Proton^{™} System, Ion S5 system and ION S5 XL system, each with different throughput characteristics (see world wide web thermofisher.com/us/en/home/life-science/sequencing/next-generation-sequencing.html). An automated library and template preparation system is also available (Ion Chef^{™}). A large number of applications are supported, including targeted and de novo DNA and RNA sequencing, transcriptome sequencing, microbial sequencing, copy number variation detection, small RNA and miRNA sequencing and CHIP-seq (chromatin immunoprecipitation sequencing).

Illumina sequencing is based on a technique known as bridge amplification in which nucleic acids with appropriate adapters ligated on each end are used as substrates for repeated amplification synthesis reactions on a solid support (glass slide) that contains primers complementary to a ligated adapter (see, e g., Slatko et al., Curr. Protoc. Mol. Biol. 122(1), e59 (2018)). The primers on the slide are spaced such that the nucleic acids, which is then subjected to repeated rounds of amplification, creates clonal "clusters" consisting of about 1000 copies. Each glass slide can support millions of parallel cluster reactions. During the synthesis reactions, proprietary modified nucleotides, corresponding to each of the four bases, each with a different fluorescent label, are incorporated and then detected. The nucleotides also act as terminators of synthesis for each reaction, which are unblocked after detection for the next round of synthesis.

Ultima Genomics has developed a sequencing-by-synthesis technology for clonal colonies of a target nucleic acid situated on a rotating platform. The target nucleic acid molecules are annealed with a primer, which can be extended with a mixture of standard and terminating nucleotides. The rotation of the platform can be configured to coordinate scanning cycles with extension cycles (see e.g., US20190153520, US20190153531).

Other sequencing methods include, for example, Sanger sequencing, single molecule real time sequencing (Pac-Bio), ONT-sequencing, exon sequencing, electron microscopy-based sequencing, panel sequencing, transistor-mediated sequencing direct sequencing, random shotgun sequencing, whole genome sequencing, capillary electrophoreses, gel electrophoresis, duplex sequencing, cycle sequencing, co-amplification at lower denaturation temperature-PCT (COLD-PCR), sequencing by reversible dye terminator, paired-end sequencing, near-term sequencing, exonuclease sequencing, sequencing by ligation, short-read sequencing, nanopore sequencing, semiconductor sequencing, sequencing-by-ligation, sequencing-by-hybridization, RNA-Seq (Illumina), Digital Gene Expression (Helicos), next generation sequencing, single molecule sequencing by synthesis (SMSS) (Helicos), massively-parallel sequencing, shotgun sequencing, Oxford Nanopore, Roche Genia, Maxim-Gilbert sequencing, primer walking, SOLiD, MS-PET sequencing or Nanopore platforms, and combinations thereof. Sequencing reactions can be performed in a variety of sample processing units, which may multiple lanes, multiple channels, multiple wells, or other mean of processing multiple sample sets substantially simultaneously. Sample processing unit can also include multiple sample chambers to enable processing of multiple runs simultaneously.

The sequence reactions may provide for sequence coverage of the genome of at least 5%, 10%, 15%, 20%, 25%, 30%, 40%, 50%, 60%, 70%, 80%, 90%, 95%, 99%, 99.9% or 100%. In other cases, sequence coverage of the genome may be less than 5%, 10%, 15%, 20%, 25%, 30%, 40%, 50%, 60%, 70%, 80%, 90%, 95%, 99%, 99.9% or 100%.

Simultaneous sequencing reactions may be performed using multiplex sequencing. In some cases, amplicons of sample nucleic acids may be sequenced with at least 1000, 2000, 3000, 4000, 5000, 6000, 7000, 8000, 9000, 10000, 50000, 100,000 sequencing reactions. In other cases, amplicons of sample nucleic acids may be sequenced with less than 1000, 2000, 3000, 4000, 5000, 6000, 7000, 8000, 9000, 10000, 50000, 100,000 sequencing reactions. Sequencing reactions may be performed sequentially or simultaneously. Subsequent data analysis may be performed on all or part of the sequencing reactions. In some cases, data analysis may be performed on at least 1000, 2000, 3000, 4000, 5000, 6000, 7000, 8000, 9000, 10000, 50000, 100,000 sequencing reactions. In other cases, data analysis may be performed on less than 1000, 2000, 3000, 4000, 5000, 6000, 7000, 8000, 9000, 10000, 50000, 100,000 sequencing reactions.

The sequencing method can be massively parallel sequencing, that is, simultaneously (or in rapid succession) sequencing any of at least 100, 1000, 10,000, 100,000, 1 million, 10 million, 100 million, or 1 billion different target nucleic acids.

Samples can be split into two or more aliquots before or after pooling of samples for analysis of DNA modification (see, e.g., Gouil et al., Essays Biochem. 63(6):639-648 (2019)). One aliquot of samples is treated such that unmodified nucleotides undergo substitution by a different nucleotide. For example, in sodium bisulfite sequencing unmodified cytosines can be converted to uracil, whereas methylated cytosines are unmodified. Comparison of sequencing reads from the different aliquots indicates, which cytosines were subject of modification.

### VII. Analysis and deconvolution of barcodes

Sequencing reads can be segregated according to their sample of origin by deconvolution based on sample barcodes. Sequencing reads can be segregated into families representing amplification copies of the same original molecule from the molecular barcodes, usually from a combination of upstream and downstream molecular barcodes, and sometimes the sequence of the sample nucleic acid. If unique molecular barcoding is used the molecular barcode or combination of upstream and downstream molecular barcodes is sufficient to indicate family of origin (i.e., all sequencing reads having the same combination of barcodes, optionally after allowing for correction of sequencing errors in the barcode, including complements for the opposing strand are grouped in the same family). If non-unique barcoding is used, then families are identified based on having the molecular barcodes or same combination of molecular barcodes together with a property of sameness among the sequences of sample molecules (such as same start and stop points, or same length) when aligned with a known reference sequence. The sequencing reads within the same family can include sequencing reads from either or both strands of the same original molecule.

The sequencing reads of family members can be compiled to derive consensus nucleotide(s) at specified positions or consensus sequence at some or all positions of a nucleic acid molecule in the original sample. If members of a family include sequencing reads of opposing strands, sequences of one strand can be converted to their complements for purposes of compiling and aligning all sequencing reads to derive consensus nucleotide(s) or sequences. A consensus nucleotide type at a position can be defined as the nucleotide type most frequently occupying that position among aligned sequencing reads. Likewise a consensus sequence can be defined as sequence of such consensus nucleotide types. For a nucleotide type to be called as consensus at a particular position in aligned sequencing reads, it can also be required that the nucleotide type occurs above a threshold frequency level among nucleotide types occupying that position in the aligned sequencing reads. For example, it can be required that the nucleotide type be present at that position in at least 50, 60, 70, 80 or 90% of sequencing reads. It can additionally or alternatively be required that the nucleotide type be present in at least one sequencing read of both strands of an original molecule. It can additionally or alternatively be required that the nucleotide type not be contradicted by more than a threshold number of sequencing reads of one or both strands in which the aligned position is occupied by a different nucleotide type. Optionally, positions showing discordance between paired forward and reverse sequencing reads of the same molecule can be disregarded in those reads in compiling a consensus sequence. Consensus deletions or insertions can be identified by similar analyses of representation and/or presence in both strands or substitutions.

Some families may include only a single sequencing read. In this case, this sequence can be taken as the sequence of a nucleic acid in the sample before amplification. Alternatively, families with only a single member sequence can be eliminated from subsequent analysis.

The criteria described above for identifying consensus nucleotides or sequence help filter genuine nucleotide variations from a reference sequence in original sample molecules and variations resulting from amplification or sequencing errors. Nucleic acid variations present in original sample molecules are likely to have greater representation in sequencing reads in general and particularly in sequencing reads of both strands than variations resulting from amplification or sequencing errors and thus be designated as consensus nucleotide types or sequences of such nucleotide types.

Having determined consensus nucleotides and/or consensus sequences within individual families, the results can be compiled to provide an indication of what nucleotide variations are present in a sample compared with a known reference sequence. The known reference sequence can be that of a gene, chromosome or genome among others. Such a compilation can provide an additional filter to distinguish genuine sequence variations from amplification and sequencing errors and provide an indication of the representation or allele frequency of such variations relative to wildtype in a sample. For any position of interest in a reference sequence for a sample (e.g., wildtype human genome sequence), one can determine which families have sequencing reads spanning that position. From those families one can determine a representation of variant nucleotide type, deletion or insertions, if any, and wildtype nucleotide type for that position. A variation can be called as being present at the position if the number of families including a variant nucleotide type, deletion or insertions exceeds a threshold, or the ratio of families with the variant nucleotide type, deletion or insertion to wildtype exceeds a threshold among other criteria. The ratio of variant nucleotide type, deletion or insertion to wildtype nucleotide type also provides an indication of the representation of the variant nucleotide. Such an analysis can be performed for each nucleotide of interest in a reference sequence corresponding to a particular sample, thus providing a variant profile of that sample. The analysis can be repeated for each sample using families of sequencing reads and their consensus nucleotides or nucleotide sequences derived as discussed above. Thus, each sample can be characterized by a variant nucleotide type profile.

Consensus nucleotides or sequences can also be compared across different sample aliquots subject to treatment resulting in differential substitution of modified and unmodified nucleotides, as in bisulfite analysis. Such analysis indicates which nucleotides in samples molecules are modified, such as by methylation.

Sequence families can also be used to provide an indication of copy number variation (see, e.g., WO2017/106768, WO/2015/100427). The number of families having a consensus sequencing read spanning a particular locus or within a defined window of a genome compared with the number of families mapping to a locus or window elsewhere in the genome, provides a measure of copy number variation, which can arise from either amplification or loss of an allele. Measured numbers of families can be normalized as needed to account for such factors as differences in window size, sequencing coverage or enrichment for different regions of a genome.

### VIII. Applications

Sequences and genetic variations within them determined by the present methods can be used to diagnose presence or absence of conditions, particularly cancer, in a subject, to characterize conditions (e.g., selection of appropriate treatment or staging cancer or determining heterogeneity of a cancer), monitor response to treatment of a condition, effect prognosis risk of developing a condition or subsequent course of a condition.

Various cancers may be detected using the present methods. Cancers cells, as most cells, can be characterized by a rate of turnover, in which old cells die and replaced by newer cells. Generally dead cells, in contact with vasculature in a given subject, may release DNA or fragments of DNA into the blood stream. This is also true of cancer cells during various stages of the disease. Cancer cells may also be characterized, dependent on the stage of the disease, by various genetic aberrations such as copy number variation as well as rare mutations. This phenomenon may be used to detect the presence or absence of cancers individuals using the methods described herein.

The types and number of cancers that may be detected may include blood cancers, brain cancers, lung cancers, skin cancers, nose cancers, throat cancers, liver cancers, bone cancers, lymphomas, pancreatic cancers, skin cancers, bowel cancers, rectal cancers, thyroid cancers, bladder cancers, kidney cancers, mouth cancers, stomach cancers, solid state tumors, heterogeneous tumors, homogenous tumors and the like.

Cancers can be detected from genetic variations including mutations, rare mutations, indels, copy number variations, transversions, translocations, inversion, deletions, aneuploidy, partial aneuploidy, polyploidy, chromosomal instability, chromosomal structure alterations, gene fusions, chromosome fusions, gene truncations, gene amplification, gene duplications, chromosomal lesions, DNA lesions, abnormal changes in nucleic acid chemical modifications, abnormal changes in epigenetic patterns, abnormal changes in nucleic acid methylation infection and cancer.

Genetic data can also be used for characterizing a specific form of cancer. Cancers are often heterogeneous in both composition and staging. Genetic profile data may allow characterization of specific sub-types of cancer that may be important in the diagnosis or treatment of that specific sub-type. This information may also provide a subject or practitioner clues regarding the prognosis of a specific type of cancer and allow either a subject or practitioner to adapt treatment options in accord with the progress of the disease. Some cancers progress, becoming more aggressive and genetically unstable. Other cancers may remain benign, inactive or dormant. The system and methods of this disclosure can be useful in determining disease progression.

The present methods are also useful in determining the efficacy of particular treatment options. For example, the number of variations detected, irrespective of their precise identity, is a predictor of amenability to immunotherapy because the mutations create neoepitopes that can be subject of immune attack (see e.g., US20200370129).

Other variations or copy number variations indicate suitability of a particular drug. Some examples of such variations are as follows:

| **Gene** | **Variation** | **Cancer** | **Drug** |
|---|---|---|---|
| EGFR/ErbB 1 | Mutations (e.g. L858R, ex19del, T790M) | NSCLC | gefitinib, erlotinib, afatinib, osimertinib, dacomitinib |
| HER2/ErbB2 | Amplification | Breast | trastuzumab, T-DM1, trastuzumab + pertuzumab, lapatinib, neratinib |
| | Amplification | Esophagogastric | trastuzumab |
| | Point mutations (V659E) | NSCLC | lapatinib |
| c-Met | ex14 skipping mutations, amplification | NSCLC | crizotinib, capmatinib, savolitinib*, tepotinib |
| RET | Fusion | NSCLC | selpercatinib, pralsetinib, cabozantinib, 3A vandetanib |
| ALK | Fusion | NSCLC | crizotinib, alectinib, ceritinib, lorlatinib brigatinib |
| | Mutations (L1196M, L1196Q) | Soft tissue sarcoma | crizotinib, ceritinib |
| ROS1 | Fusion, mutation | NSCLC | crizotinib, entrectinib |
| NTRK | Fusion | All tumors | larotrectinib, entrectinib |
| c-Kit | Mutations (e.g. 449_514mut), deletions (e.g. D419del) | GIST | imatinib, sunitinib, regorafenib, sorafenib |
| | | Thymic tumors | sunitinib |
| | Mutations (e.g. K642E) | Melanoma | imatinib |
| PDGFR | Mutations (e.g. D842V), deletions (e.g. C456_N468del) | GIST | imatinib, dasatinib |
| | | Leukemia, myelodysplasia | imatinib |
| FGFR1 | Amplification | LSCC | erdafitinib |
| | | NSCLC | AZD4547 |

| Gene | **Variation** | **Cancer** | **Drug** |
|---|---|---|---|
| FGFR2 | Fusion, mutation | Bladder, cholangiocarcinoma | erdafitinib, pemigatinib |
| | Amplification | Breast | dovitinib |
| FGFR3 | Fusion, mutation | Bladder | erdafitinib |
| RAS | Wild-type | CRC | cetuximab, panitumumab |
| BRAF | Mutations (e.g. V600E) | Melanoma | vemurafenib, dabrafenib, trametinib, trametinib |
| | | NSCLC | dabrafenib + trametinib |
| | | Histiocytosis | cobimetinib |
| | Mutation (V600E) | CRC | encorafenib + cetuximab |
| | Fusions | Ovarian | trametinib, cobimetinib |
| MEK | Mutations | Melanoma, NSCLC, ovarian, histiocytic disorder | trametinib, cobimetinib, selumetinib |
| mTOR | Mutations (e.g. E2014K) | Bladder, RCC | everolimus, temsirolimus |
| AKT | Mutation (E17K) | Breast, ovarian | capivasertib |
| PTEN | Homozygous deletions, loss-of-function mutations | Breast | capivasertib |
| PIK3CA | Mutations | Breast | alpelisib |
| CDK4 | Amplification | Soft tissue sarcoma | palbociclib |
| IDH1 | Mutations | AML, cholangiocarcinoma | ivosidenib |
| IDH2 | Mutations | AML | enasidenib |
| BRCA1/2 and ATM | Mutations (somatic) | Breast | olaparib, talazoparib, rucaparib |
| | Mutations (somatic) | Ovarian, prostate | rucaparib, olaparib |
| ERα | Mutations (e.g. E380Q) | Breast | fulvestrant |
| MSI-H | Not applicable | All | pembrolizumab |
| TML | Not applicable | Multiple tumor types | pembrolizumab, nivolumab |

The present methods can also be used to monitor therapy. For example, a successful treatment can initially be associated with an increase in nucleotide or copy number variations in cell free DNA as cancer cells die and release their DNA to the circulation. This initial increase can be followed by a decrease reflecting fewer if any remaining cancer cells to release their DNA. There can also be a subsequent increase in nucleotide or copy number variations following a period of remission providing an indication of recurrence of the cancer.

The present methods can also be used for detecting genetic variations in conditions other than cancer. Immune cells, such as B cells, undergo copy number variation associated with certain diseases. Clonal expansions can be monitored using copy number variation detection as a measure of disease progression. The present methods may be used to determine or profile rejection activities of the host body, as immune cells attempt to destroy transplanted tissue to monitor the status of transplanted tissue as well as altering the course of treatment or prevention of rejection. Copy number variation or variant nucleotide can be used to determine how a population of pathogens are changing during the course of infection. For example during chronic infections, such as HIV/AIDs or Hepatitis infections, y viruses may change life cycle state and/or mutate into more virulent forms during the course of infection.

The present methods can be used to generate or profile, fingerprint or set of data that is a summation of genetic information derived from different cells in a heterogeneous disease. This set of data may comprise copy number variation and nucleotide variation or both.

The present methods can be used to diagnose, prognose, monitor or observe cancers or other diseases of fetal origin. That is, these methodologies can be employed in a pregnant subject to diagnose, prognose, monitor or observe cancers or other diseases in a unborn subject whose DNA and other nucleic acids may co-circulate with maternal molecules.

### I. Kits

Any or all of the reagents described for performing the above methods can be include in a kit. Some such kits include a primer with a 3' target binding region and a 5' stem loop region as previously described. Such kits can also include a loop primer, one or more adapters for linkage to a target nucleic acid and a reverse sequencing primer, each as described above. As described above, the methods use 5' and 3' adapters flanking a target nucleic acid, which include different primer binding sites. Other kits include a primer with two 3' target binding regions separated by an intervening region. Such primers can also include one or more adapters and a reverse sequencing primer as described above.

The adapters in such kits can be supplied separately, e.g., a 5' and a 3' adapter. Alternatively, a single adapter (with the possible exception of barcode region(s)) with asymmetric single-stranded regions can be used, e.g., Y-shaped, stem-hairpin or bubble. Such an adapter when flanking both ends of a double-stranded target nucleic acid is disposed to flank the individual strands of the target nucleic acid with the different single-sequences in the adapter. These single-stranded sequences flanking a single stranded target nucleic acid can be considered to be 5' and 3' adapters.

Such kits can also include a polymerase, optionally, a strand displacing polymerase, and nucleotide triphosphates for extension. Such a kit can include a phosphatase for removing a phosphate blocking group from a primer. Such nucleotide triphosphates or at least some of them can be labelled and/or some can be nucleotide analogs resulting in chain termination. Thus, for example, such a kit can include a set of standard nucleotide triphosphates and a set of labelled nucleotide triphosphates and/or chain terminator nucleotide triphosphates. Such a kit can also include a label or other instructions for performing the methods, which can be provided in hard form, such as paper, or soft form for display on a computer monitor, or the like.

All publications, patents and patent applications, accession numbers, websites and the like mentioned in this specification are incorporated by reference to the same extent as if each individual publication, patent or patent application was so individually denoted. To the extent more different content is associate with an accession number or other reference at different times, the content in effect as of the effective filing date of this application is meant. The effective filing date is the date of the earliest priority application disclosing the accession number in question. Unless otherwise apparent from the context any element, embodiment, step, feature or aspect of the invention can be performed in combination with any other.

### EXAMPLES

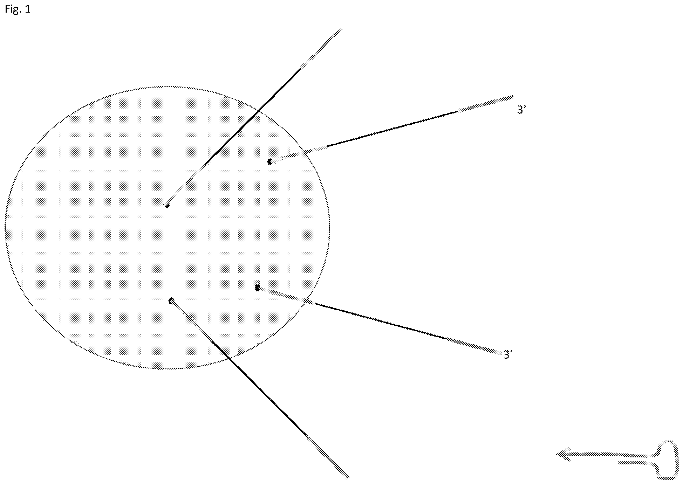

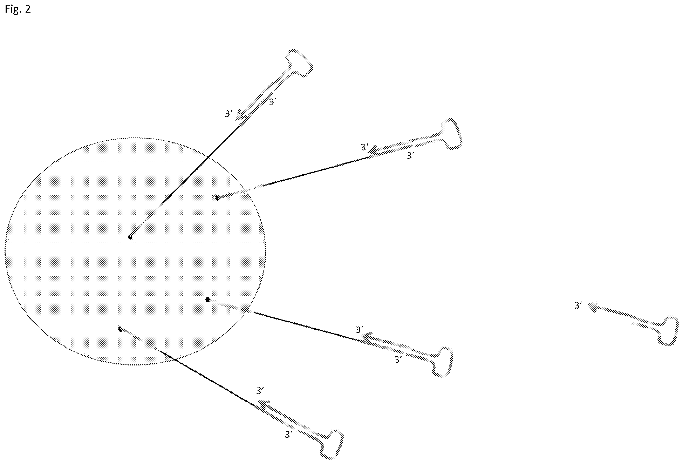

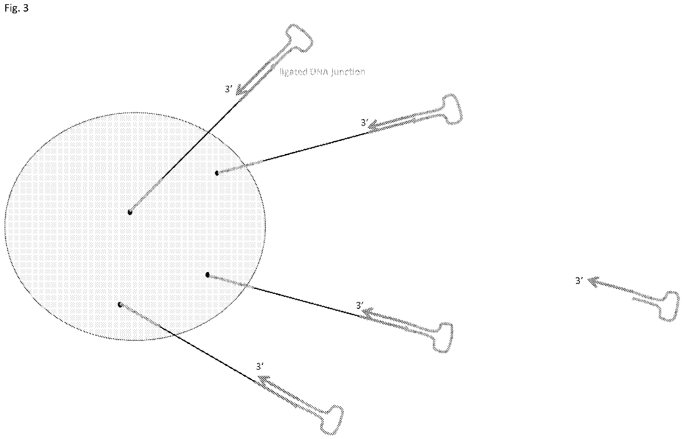

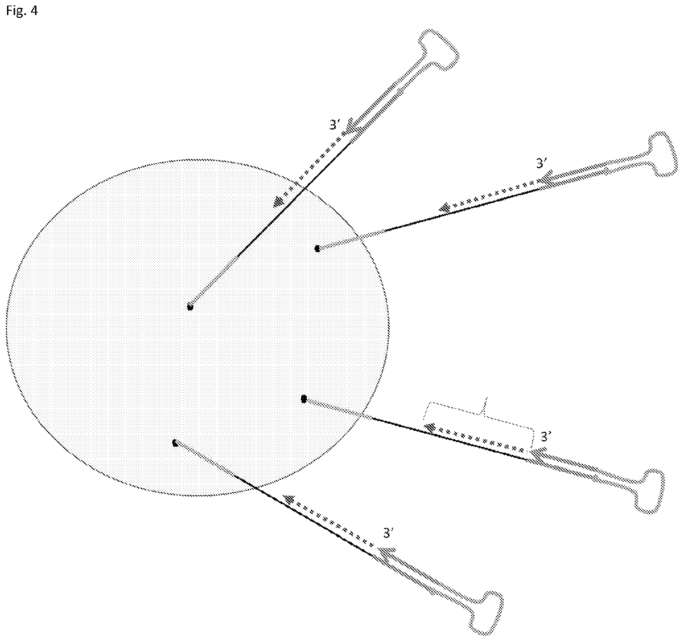

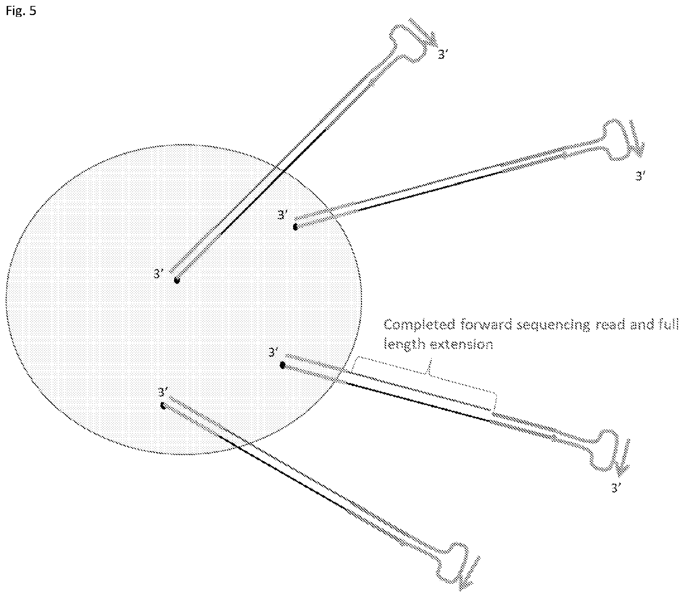

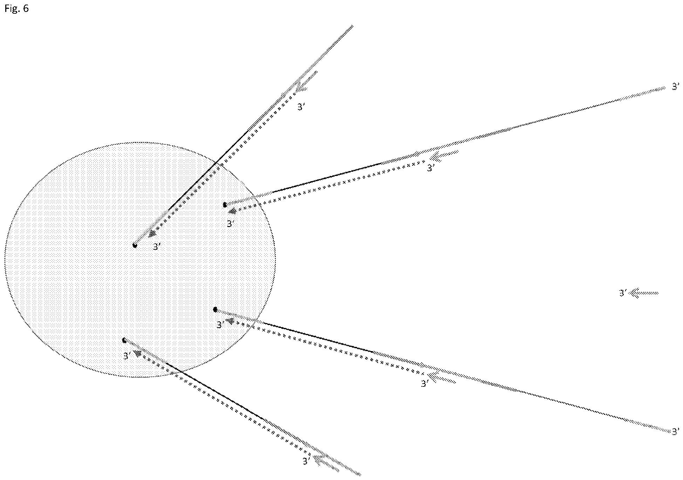

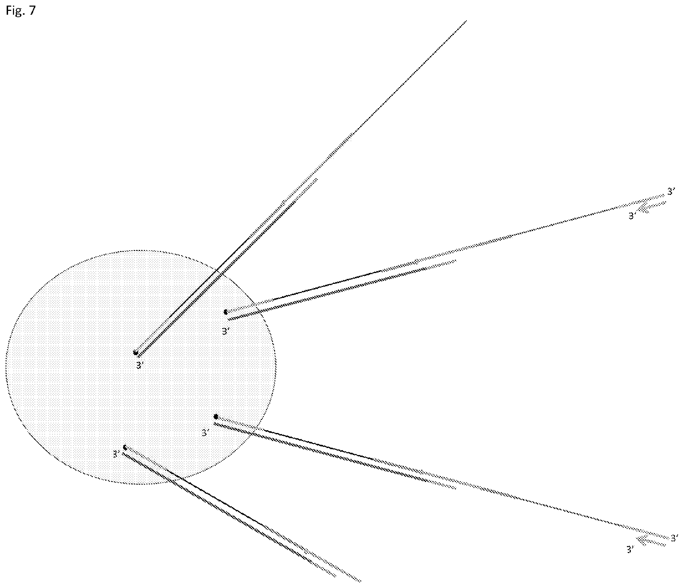

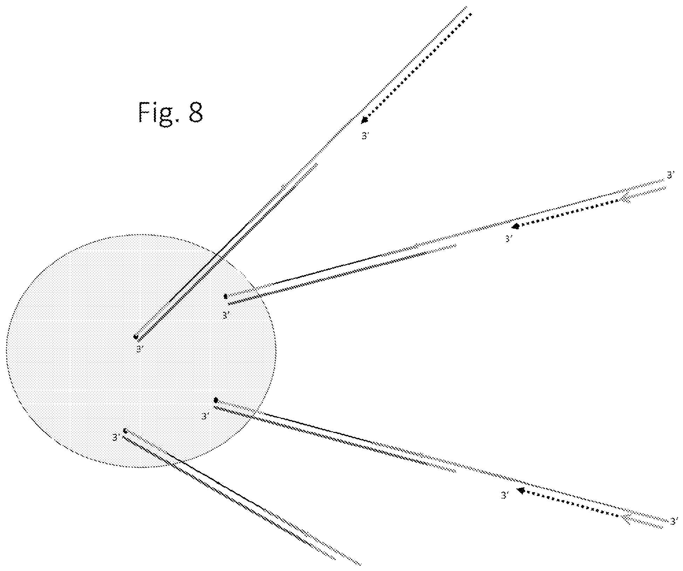

An exemplary form of the method is shown in Fig. 1 Fig. 1 at left shows a product of emulsion PCR, that is, single stranded target DNA molecules flanked by a pair of adapters. The single strandedsingle-stranded DNA molecules attached to the same bead are clonal amplicons of the same original molecule. The beads are initially in separate cells within an emulsion but the emulsion can be broken mixing the beads before proceeding further. The right of Fig. 1 shows a stem loop (or hairpin primer). A 3' portion of the primer is a target binding region configured to bind to the adapter at the 3' end of DNA molecules attached to beads. The 5' portion of the primer is a stem loop. Fig. 2 shows the stem loop primer annealed to the adapter at the 3' end of DNA molecules. Fig. 3 shows ligation of the 5' end of the stem loop primer to the 3' end of DNA molecules attached to the bead. The ligation can alternatively be performed after forward sequencing. Fig. 4 shows extension of the 3' end of the stem loop primer in a sequencing by synthesis reaction to generate a complementary strand and provide a forward sequencing read. Fig. 5 shows annealing of a primer to the loop region of the stem loop primer. Fig. 6 shows extension from the primer to the loop region. The extension displaces the complementary strand synthesized previously be extension of the 3' end of the stem loop primer. Fig. 7 shows a reverse read primer hybridizing to the 3' adapter region of the complementary strand. Fig. 8 shows extension of the reverse read primer with the complementarity strand acting as a template to generate a reverse strand sequencing read.

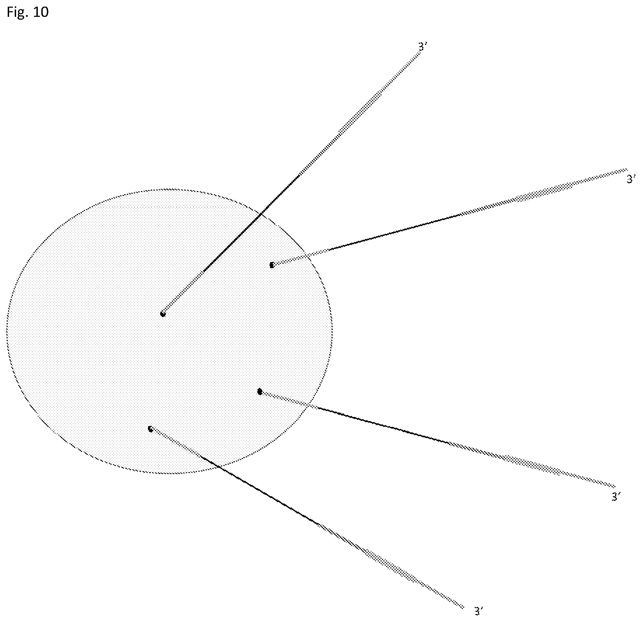

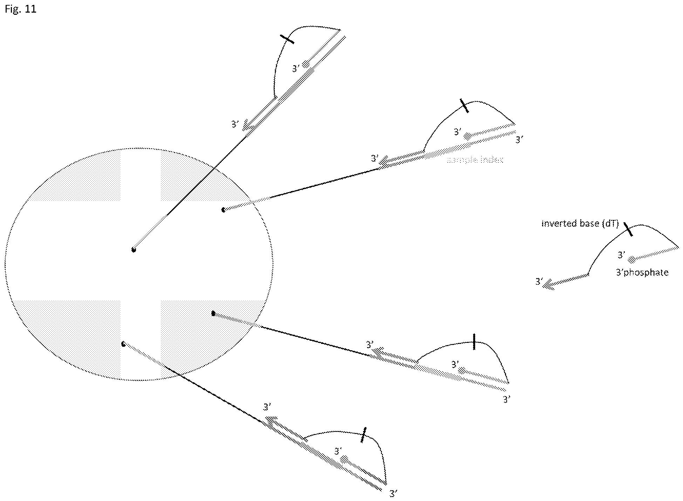

Fig. 10 shows a similar arrangement to Fig. 1 but in this case the adapter at the 3' end of the target nucleic acid has two primer binding sites separated by a sample index. The primer binding site proximal to the 3' end of the target DNA molecule is referred to as a forward primer binding site, and the primer binding site distal to the 3' end of the target DNA molecule (i.e., 3' to the sample index in Fig. 10) is referred to as an index primer binding site. Inclusion of a sample index between the primer binding sites is optional. Fig. 11 shows a dual functional primer including two target binding regions configured to duplex with the forward primer binding site and index primer binding site in the adapter of the target nucleic acid. The target binding region complementary to the index primer binding site can be blocked at its 3' end, e.g., with a phosphate group to prevent extension until required. Other blocking moieties that can be used include an ammonium group, a modified nucleotide, e.g., a 3' 2' dideoxynucleotide or 3' deoxyadenosine 5'-triphosphate (cordycepin or a short nucleotide sequence having a 3'-to-5' orientation, so that there is no free hydroxyl group at the 3'-terminus, the use of a 3' alkyl group, a 3' non-nucleotide moiety), phosphorothioate, alkane-diol residues, peptide nucleic acid (PNA), nucleotide residues lacking a 3' hydroxyl group at the 3'-terminus, or a nucleic acid binding protein (see, e.g., Arnold et al., "Non-Nucleotide Linking Reagents for Nucleotide Probes," U.S. Pat. No. 6,031,091).

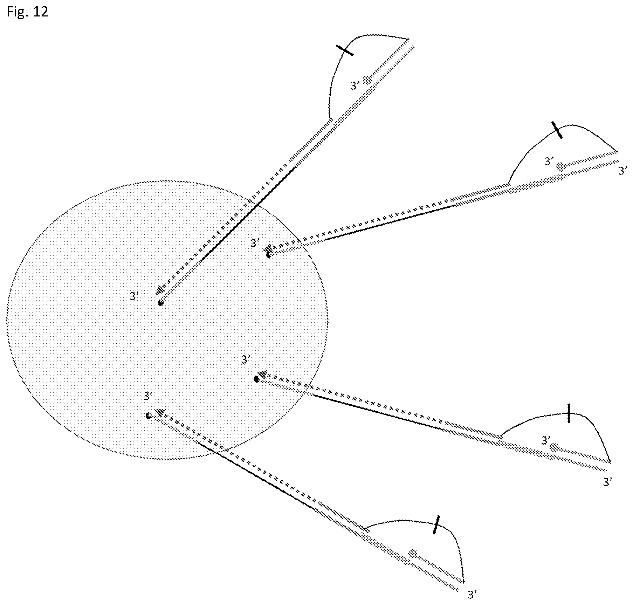

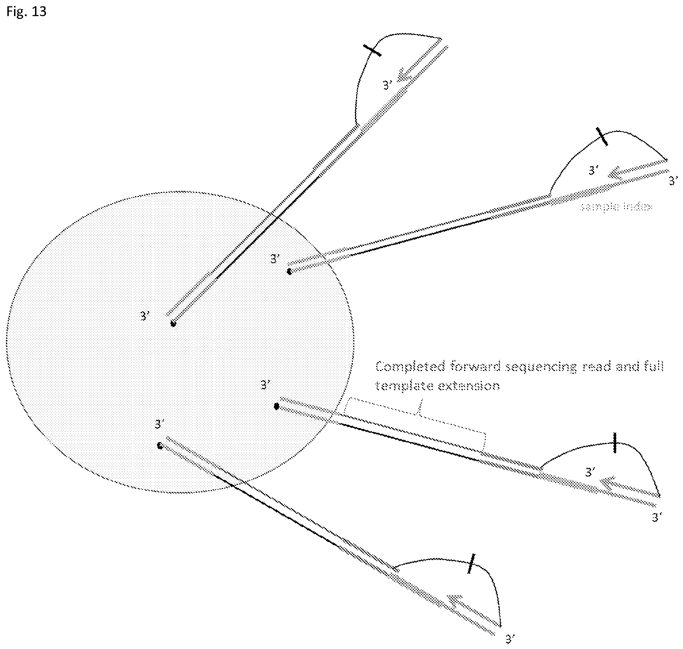

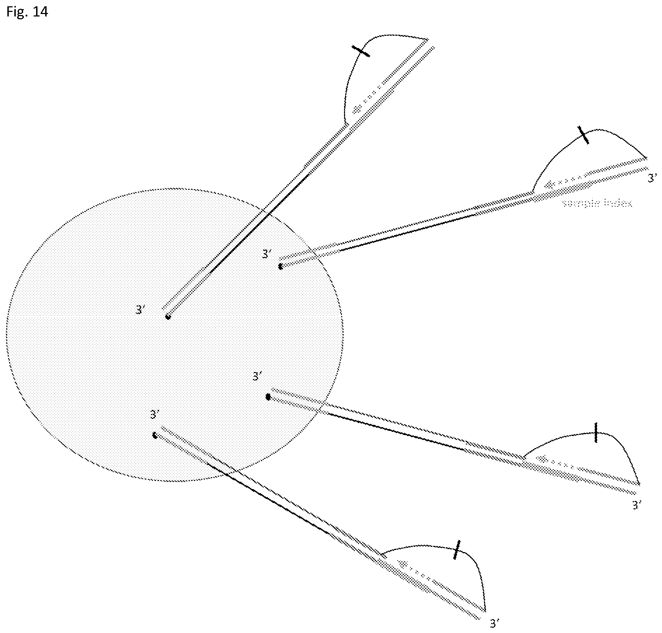

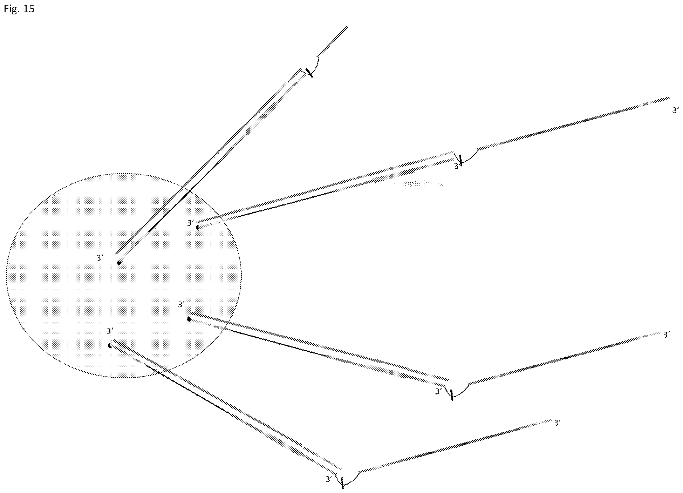

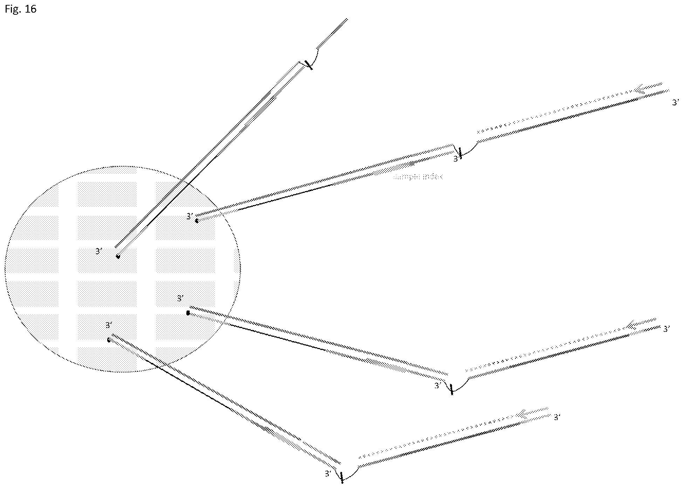

Each of the target binding regions has a free 3' end to allow extension from the primer. The target binding regions flank an intervening region, which includes an inversion modification, such as an inverted nucleotide to permit the target binding regions to be in opposing orientations and thus both have free 3' ends. The left portion of the figure shows the dual functional primer with both target binding sites duplexed with the corresponding primer binding sites on the adapter. Fig. 12 shows extension from the target binding region duplexed with the forward primer binding site. The extension can be a sequencing by synthesis reaction in which the identity of added nucleotides is detected successively generating a forward sequencing read or can be an extension reaction without sequencing. Following synthesis of a complementary strand to the single-stranded target nucleic acid, phosphate blockage of the 3' terminus of the index target binding site can be removed by kinase treatment (Fig. 13). Fig. 14 shows extension from the target binding site duplexed with the index primer binding site. If this target binding region is initially blocked to prevent premature extension, the block can be removed, e.g., with phosphatase treatment for a phosphate block. The extension can provide a read of the sample index. The extension also generates a further complementary strand to the initially single-stranded target nucleic acid displacing the original complementary strand as shown in Fig. 15. Extension can be with strand-displacing polymerase. Alternatively extension can be with a non-strand displacing polymerase having 5'-3' exonuclease activity (e.g., Taq) in which case blockage of the 3' terminal and removal prior to extension is not needed. The original complementary strand is still tethered to the bead via the dual functional primer and original target nucleic acid. Fig. 16 shows a reverse primer that binds to the adapter at the 3' end of the displaced complementary strand. Extension from the reverse primer in a sequencing by synthesis reaction generates a reverse strand sequencing read.

In a variation, a forward sequencing read is generated primed from a conventional primer binding to a forward primer binding site in an adapter of an adapter flanked single-stranded target molecule, such as shown in Fig. 1 or Fig. 10. The complementary strand generated by the forward sequencing read is then displaced restoring the original single-stranded target molecule as shown in Fig. 1 or Fig. 10. The method then proceeds as previously described with stem loop primer or the dual functional primer except that the extension for the target binding region duplexed with the forward primer binding site is an extension without generating a sequencing read (sometimes referred to as a dark extension).

### NUMBERED EMBODIMENTS

1. A method of obtaining paired sequencing reads of a target nucleic acid, comprising
   (a) contacting a single-stranded target nucleic acid flanked at its 5' end by a 5' adapter immobilized to a support and at its 3' end by a 3' adapter with a forward primer comprising a 3' target binding region and a 5' stem-loop to form a reaction mix, wherein the target binding region binds the 3' adapter;
   (b) ligating a free 5' end of the stem loop to the 3' adapter;
   (c) conducting an extension reaction, wherein the target binding region primes synthesis of a first complementary strand to the target nucleic acid and optionally provides a sequencing read of the target nucleic acid;
   (d) contacting the reaction mix with a loop primer that anneals to the loop region of the stem loop;
   (e) conducting an extension reaction, wherein the loop primer primes synthesis of a second complementary strand to the target nucleic acid, and the first complementary strand is displaced from duplexing with the target nucleic acid;
   (f) contacting the reaction mix with a reverse primer, which anneals to a complement of the 5' adapter at the 3' end of the displaced first complementary strand; and
   (g) conducting a sequencing-by-extension reaction, wherein the reverse primer primes synthesis of a strand complementary to the displaced first complementary strand and provides a sequencing read of the displaced first complementary strand.
2. The method of embodiment 1, further comprising before step (a) contacting the single-stranded target nucleic acid with a second forward primer comprising a target binding region, which binds to a complementary site in the 3' adapter;
   conducting a sequencing-by-extension reaction wherein the target binding region of the second forward primer primes synthesis of a further complementary strand of the target nucleic acid and provides a sequencing read of the target nucleic acid; and
   displacing the further complementary strand from duplexing with the target nucleic acid.
3. The method of embodiment 1, wherein step (c) is a sequencing-by-extension reaction that provides a sequence read of the target nucleic acid.
4. The method of embodiment 1, wherein step (c) is performed before step (b).
5. A method of obtaining paired sequencing reads of a target nucleic acid, comprising
   (a) contacting a single-stranded target nucleic acid flanked at its 5' end by a 5' adapter immobilized to a support and at its 3' end by a 3' adapter with a forward primer comprising a 3' target binding region and a 5' stem loop to form a reaction mix, wherein the target binding region binds the 3' adapter and wherein the target binding region comprises a cleavable site;
   (b) ligating a free 5' end of the stem loop to the 3' adapter;
   (c) conducting a sequencing-by-extension reaction, wherein the target binding region primes synthesis of a first complementary strand to the target nucleic acid and provides a sequencing read of the target nucleic acid; cleaving the cleavable site, and conducting a further extension reaction wherein the remainder of the target binding region after cleavage primes synthesis of a further complementary strand to the target nucleic acid;
   (d) contacting the reaction mix with a loop primer that anneals to the loop region of the stem loop;
   (e) conducting an extension reaction wherein the loop primer primes synthesis of a second complementary strand to the target nucleic acid, which displaces the further complementary strand from duplexing with the target nucleic acid;
   (f) contacting the reaction mix with a reverse primer, which anneals to a complement of the 5' adapter at the 3' end of the displaced further complementary strand;
   (g) conducting a sequencing-by-extension reaction, wherein the reverse primer primes synthesis of a strand complementary to the displaced further complementary strand and provides a sequencing read of the displaced further complementary strand.
6. A method of obtaining paired sequencing reads from a target nucleic acid, comprising
   (a) contacting a single-stranded target nucleic acid flanked at its 5' end by a 5' adapter immobilized to a support and at its 3' end by a 3' adapter with a forward hairpin primer comprising a 3' target binding region and a 5' stem loop and a forward linear primer to form a reaction mix, wherein the target binding region of the forward hairpin stem primer and the linear forward primer bind the 3' adapter with the stem loop primer between the linear primer and the 3' end;
   (b) ligating a free 5' end of the step loop to the 3' end of the adapter molecule at the 3' end of the target nucleic acid;
   (c) conducting a sequencing-by-extension reaction, wherein the linear primer primes synthesis of a first complementary strand to the target nucleic acid and provides a sequencing read of the target nucleic acid; and conducting a further extension reaction wherein the target binding region of the forward stem loop primer primes synthesis of a further complementary strand to the target nucleic acid;
   (d) contacting the reaction mix with a loop primer that anneals to the loop region of the stem loop;
   (e) conducting an extension reaction wherein the loop primer primes synthesis of a second complementary strand to the target nucleic acid, which displaces the further complementary strand from duplexing with the target nucleic acid;
   (f) contacting the reaction mix with a reverse primer, which anneals with a complement of the 5' adapter at the 3' end of the displaced further complementary strand;
   (g) conducting a sequencing-by-extension reaction, wherein the reverse primer primes synthesis of a strand complementary to the displaced further complementary strand and provides a sequencing read of the displaced further complementary strand.
7. The method of embodiment 6, wherein the stem loop forward primer contains an extension-blocked nucleotide and the method further comprises removing the block before extending the stem loop forward primer.
8. A method of obtaining paired sequencing reads from a target nucleic acid, comprising
   (a) contacting a single-stranded target nucleic acid flanked at its 5' end by a 5' adapter immobilized to a support and at its 3' end by a 3' adapter with a forward primer comprising first and second 3' target binding regions flanking an intervening region to form a reaction mix, wherein the first and second target binding regions bind complementary sites in the 3' adapter molecule, the respective sites being distal and proximal to the 3' end of the 3' adapter;
   (b) conducting an extension reaction, wherein the first target binding region primes synthesis of a first complementary strand to the target nucleic acid and optionally provides a sequencing read of the target nucleic acid;
   (c) conducting a second extension reaction wherein the second target binding region primes synthesis of a second complementary strand to the target nucleic acid, which displaces the complementary strand from duplexing with the target nucleic acid;
   (d) contacting the reaction mix with a reverse primer, which anneals with a complement of the 5' adapter at the 3' end of the displaced first complementary strand; and
   (e) conducting a sequencing-by-extension reaction, wherein the reverse primer primes synthesis of a strand complementary to the displaced first complementary strand and provides a sequencing read of the displaced first complementary strand.
9. The method of embodiment 8, further comprising before step (a) contacting the single-stranded target nucleic acid with a second forward primer comprising a target binding region, which binds to a complementary site in the adapter molecule at the 3' end of the target nucleic acid;
   conducting a sequencing-by-extension reaction wherein the target binding region of the second forward primer primes synthesis of a further complementary strand of the target nucleic acid and provides a sequencing read of the target nucleic acid; and
   displacing the further complementary strand from duplexing with the target nucleic acid.
10. The method of embodiment 8, wherein the intervening region comprises an inverted nucleotide.
11. The method of embodiment 8, wherein the intervening region comprises a non-nucleotide linkage.
12. The method of embodiment 8, wherein the second target binding region is extension blocked and the method further comprises removing the block to extension before performing the extension primed by the second target binding region.
13. The method of embodiment 12, wherein the second target binding region is extension blocked by a 3' terminal phosphate group and removing the block comprising treating with a phosphatase to remove the phosphate.
13. The method of embodiment 8, wherein the second extension is performed with a strand-displacing polymerase.
14. The method of embodiment 8, wherein the 3' adapter further comprises a sample index between the complementary sites for the first and second target binding regions.
15. The method of any preceding embodiment, wherein the 5' or 3' adapter or both further comprises a molecular index.
16. The method of any preceding embodiment wherein the 5' or 3' adapter or both further comprises a sample index.
17. The method any preceding embodiment, wherein the single-stranded nucleic acid flanked by the 5' and 3' adapters is produced by emulsion amplification.
18. The method of embodiment 17, further comprising breaking the emulsion before performing step (a).
19. The method of embodiment 17, wherein the support to which the single-stranded target nucleic acid is immobilized was used in emulsion PCR to generate the single-stranded target nucleic acid flanked by 5' and 3' adapters.
20. The method of embodiment 17, wherein the support to which the single-stranded target nucleic acid is immobilized was attached after emulsion PCR.
21. The method of any preceding embodiment, wherein the single-stranded target nucleic acid immobilized to a support is one of a clonal population of such target nucleic acids immobilized to the same support.
22. The method of embodiment 21, wherein the support is a bead.
23. The method of embodiment 21, wherein the support is an addressable region within an array.
24. The method of any preceding embodiment, wherein the sequencing is by 454, ion torrent or ultima sequencing.
25. The method of any preceding embodiment, wherein the sequencing is single molecule sequencing
26. The method of any one of embodiments 1-24, wherein the sequencing is clonal sequencing.
27. The method of embodiment wherein the length of the target nucleic acid exceeds the maximum read length of the sequencing method.
28. The method of embodiment 27, wherein the target nucleic acid is about 170 nucleotides and the maximum read length of the sequencing method is about 150 nucleotides of the target nucleic acid.
29. The method of any preceding embodiment, wherein the sequencing reads have a non-overlapping region.
30. The method of any preceding embodiment, further comprising determining a composite sequencing read from the forward and reverse sequencing reads in which any positions of discordance between forward and reverse sequencing reads are left open or as alternative nucleotides occupying the position in the forward and reverse sequencing reads.
31. A primer comprising first and second target binding regions each with a free 3' end separated by an inverted nucleotide.
32. A kit comprising a primer with first and second target binding regions, each with a free 3' end separated by an inverted nucleotide, first and second adapters, or a single adapter with asymmetric single-stranded regions disposed to flank individual strands of double-stranded target nucleic acid with first and second adapters, and a second primer, wherein the first and second target binding regions bind the first adapter and the second primer binds the second adapter.
33. The kit of embodiment 32, wherein one of the free 3' ends is blocked with a phosphate group and the kit further comprises a phosphatase to remove the phosphate.
34. A kit comprising a primer comprising a 3' target binding region and a 5' stem-loop, first and second adapters, or a single adapter with asymmetric single-stranded regions disposed to flank individual strands of double-stranded target nucleic acid with first and second adapters, a second primer and a third primer, wherein the 3' target binding region binds the first adapter, the second primer binds the loop region of the primer, and the third primer binds the second adapter.
35. The kit of any of embodiments 32-34 further comprising a strand displacing polymerase.

## Claims

1. A kit comprising a primer comprising a 3' target binding region and a 5' stem-loop, first and second adapters, or a single adapter with asymmetric single-stranded regions disposed to flank individual strands of double-stranded target nucleic acid with first and second adapters, a second primer and a third primer, wherein the 3' target binding region binds the first adapter, the second primer binds the loop region of the primer, and the third primer binds the second adapter; optionally wherein the kit further comprises a strand displacing polymerase.

2. A method of obtaining paired sequencing reads of a target nucleic acid, comprising:
(a) contacting a single-stranded target nucleic acid flanked at its 5' end by a 5' adapter immobilized to a support and at its 3' end by a 3' adapter with a forward primer comprising a 3' target binding region and a 5' stem-loop to form a reaction mix, wherein the target binding region binds the 3' adapter;
(b) ligating a free 5' end of the stem loop to the 3' adapter;
(c) conducting an extension reaction, wherein the target binding region primes synthesis of a first complementary strand to the target nucleic acid and optionally provides a sequencing read of the target nucleic acid;
(d) contacting the reaction mix with a loop primer that anneals to the loop region of the stem loop;
(e) conducting an extension reaction, wherein the loop primer primes synthesis of a second complementary strand to the target nucleic acid, and the first complementary strand is displaced from duplexing with the target nucleic acid;
(f) contacting the reaction mix with a reverse primer, which anneals to a complement of the 5' adapter at the 3' end of the displaced first complementary strand; and
(g) conducting a sequencing-by-extension reaction, wherein the reverse primer primes synthesis of a strand complementary to the displaced first complementary strand and provides a sequencing read of the displaced first complementary strand.

3. The method of claim 2:
(i) further comprising before step (a) contacting the single-stranded target nucleic acid with a second forward primer comprising a target binding region, which binds to a complementary site in the 3' adapter;
conducting a sequencing-by-extension reaction wherein the target binding region of the second forward primer primes synthesis of a further complementary strand of the target nucleic acid and provides a sequencing read of the target nucleic acid; and
displacing the further complementary strand from duplexing with the target nucleic acid;
(ii) wherein step (c) is a sequencing-by-extension reaction that provides a sequence read of the target nucleic acid; or
(iii) wherein step (c) is performed before step (b).

4. A method of obtaining paired sequencing reads of a target nucleic acid, comprising
(a) contacting a single-stranded target nucleic acid flanked at its 5' end by a 5' adapter immobilized to a support and at its 3' end by a 3' adapter with a forward primer comprising a 3' target binding region and a 5' stem loop to form a reaction mix, wherein the target binding region binds the 3' adapter and wherein the target binding region comprises a cleavable site;
(b) ligating a free 5' end of the stem loop to the 3' adapter;
(c) conducting a sequencing-by-extension reaction, wherein the target binding region primes synthesis of a first complementary strand to the target nucleic acid and provides a sequencing read of the target nucleic acid; cleaving the cleavable site, and conducting a further extension reaction wherein the remainder of the target binding region after cleavage primes synthesis of a further complementary strand to the target nucleic acid;
(d) contacting the reaction mix with a loop primer that anneals to the loop region of the stem loop;
(e) conducting an extension reaction wherein the loop primer primes synthesis of a second complementary strand to the target nucleic acid, which displaces the further complementary strand from duplexing with the target nucleic acid;
(f) contacting the reaction mix with a reverse primer, which anneals to a complement of the 5' adapter at the 3' end of the displaced further complementary strand;
(g) conducting a sequencing-by-extension reaction, wherein the reverse primer primes synthesis of a strand complementary to the displaced further complementary strand and provides a sequencing read of the displaced further complementary strand.

5. A method of obtaining paired sequencing reads from a target nucleic acid, comprising
(a) contacting a single-stranded target nucleic acid flanked at its 5' end by a 5' adapter immobilized to a support and at its 3' end by a 3' adapter with a forward hairpin primer comprising a 3' target binding region and a 5' stem loop and a forward linear primer to form a reaction mix, wherein the target binding region of the forward hairpin stem primer and the linear forward primer bind the 3' adapter with the stem loop primer between the linear primer and the 3' end;
(b) ligating a free 5' end of the step loop to the 3' end of the adapter molecule at the 3' end of the target nucleic acid;
(c) conducting a sequencing-by-extension reaction, wherein the linear primer primes synthesis of a first complementary strand to the target nucleic acid and provides a sequencing read of the target nucleic acid; and conducting a further extension reaction wherein the target binding region of the forward stem loop primer primes synthesis of a further complementary strand to the target nucleic acid;
(d) contacting the reaction mix with a loop primer that anneals to the loop region of the stem loop;
(e) conducting an extension reaction wherein the loop primer primes synthesis of a second complementary strand to the target nucleic acid, which displaces the further complementary strand from duplexing with the target nucleic acid;
(f) contacting the reaction mix with a reverse primer, which anneals with a complement of the 5' adapter at the 3' end of the displaced further complementary strand;
(g) conducting a sequencing-by-extension reaction, wherein the reverse primer primes synthesis of a strand complementary to the displaced further complementary strand and provides a sequencing read of the displaced further complementary strand,
optionally wherein the stem loop forward primer contains an extension-blocked nucleotide and the method further comprises removing the block before extending the stem loop forward primer.

6. A method of obtaining paired sequencing reads from a target nucleic acid, comprising
(a) contacting a single-stranded target nucleic acid flanked at its 5' end by a 5' adapter immobilized to a support and at its 3' end by a 3' adapter with a forward primer comprising first and second 3' target binding regions flanking an intervening region to form a reaction mix, wherein the first and second target binding regions bind complementary sites in the 3' adapter molecule, the respective sites being distal and proximal to the 3' end of the 3' adapter;
(b) conducting an extension reaction, wherein the first target binding region primes synthesis of a first complementary strand to the target nucleic acid and optionally provides a sequencing read of the target nucleic acid;
(c) conducting a second extension reaction wherein the second target binding region primes synthesis of a second complementary strand to the target nucleic acid, which displaces the complementary strand from duplexing with the target nucleic acid;
(d) contacting the reaction mix with a reverse primer, which anneals with a complement of the 5' adapter at the 3' end of the displaced first complementary strand; and
(e) conducting a sequencing-by-extension reaction, wherein the reverse primer primes synthesis of a strand complementary to the displaced first complementary strand and provides a sequencing read of the displaced first complementary strand.

7. The method of claim 6:
(i) further comprising before step (a) contacting the single-stranded target nucleic acid with a second forward primer comprising a target binding region, which binds to a complementary site in the adapter molecule at the 3' end of the target nucleic acid;
conducting a sequencing-by-extension reaction wherein the target binding region of the second forward primer primes synthesis of a further complementary strand of the target nucleic acid and provides a sequencing read of the target nucleic acid; and
displacing the further complementary strand from duplexing with the target nucleic acid;
(ii) wherein the intervening region comprises an inverted nucleotide;
(iii) wherein the intervening region comprises a non-nucleotide linkage;
(iv) wherein the second target binding region is extension blocked and the method further comprises removing the block to extension before performing the extension primed by the second target binding region, optionally wherein the second target binding region is extension blocked by a 3' terminal phosphate group and removing the block comprising treating with a phosphatase to remove the phosphate;
(v) wherein the second extension is performed with a strand-displacing polymerase; or
(vi) wherein the 3' adapter further comprises a sample index between the complementary sites for the first and second target binding regions.

8. The method of any one of claims 2 to 7, wherein the 5' or 3' adapter or both further comprises a molecular index and/or a sample index.

9. The method any one of claims 2 to 8, wherein the single-stranded nucleic acid flanked by the 5' and 3' adapters is produced by emulsion amplification, optionally:
(i) further comprising breaking the emulsion before performing step (a);
(ii) wherein the support to which the single-stranded target nucleic acid is immobilized was used in emulsion PCR to generate the single-stranded target nucleic acid flanked by 5' and 3' adapters; or
(iii) wherein the support to which the single-stranded target nucleic acid is immobilized was attached after emulsion PCR.

10. The method of any one of claims 2 to 9, wherein the single-stranded target nucleic acid immobilized to a support is one of a clonal population of such target nucleic acids immobilized to the same support, optionally wherein the support is a bead or an addressable region within an array.

11. The method of any one of claims 2 to 10, wherein the sequencing is: (i) by 454, ion torrent or ultima sequencing; and/or (ii) single molecule sequencing or clonal sequencing.

12. The method of any one of claims 2 to 11, wherein the length of the target nucleic acid exceeds the maximum read length of the sequencing method, optionally wherein the target nucleic acid is about 170 nucleotides and the maximum read length of the sequencing method is about 150 nucleotides of the target nucleic acid.

13. The method of any one of claims 2 to 12:
(i) wherein the sequencing reads have a non-overlapping region; and/or
(ii) further comprising determining a composite sequencing read from the forward and reverse sequencing reads in which any positions of discordance between forward and reverse sequencing reads are left open or as alternative nucleotides occupying the position in the forward and reverse sequencing reads.

14. A primer comprising first and second target binding regions each with a free 3' end separated by an inverted nucleotide.

15. A kit comprising a primer with first and second target binding regions, each with a free 3' end separated by an inverted nucleotide, first and second adapters, or a single adapter with asymmetric single-stranded regions disposed to flank individual strands of double-stranded target nucleic acid with first and second adapters, and a second primer, wherein the first and second target binding regions bind the first adapter and the second primer binds the second adapter, optionally wherein:
(i) one of the free 3' ends is blocked with a phosphate group and the kit further comprises a phosphatase to remove the phosphate and/or
(ii) the kit further comprises a strand displacing polymerase.
